(19) 

Europäisches
Patentamt
European
Patent Office
Office européen
des brevets

(11)   **EP 4 495 258 A1**

(12)   **EUROPEAN PATENT APPLICATION**
published in accordance with Art. 153(4) EPC

(43) Date of publication:
**22.01.2025   Bulletin 2025/04**

(21) Application number: **22932422.3**

(22) Date of filing: **29.12.2022**

(51) International Patent Classification (IPC):
**C12Q 1/6883** (2018.01)

(52) Cooperative Patent Classification (CPC):
**C12Q 1/6883**

(86) International application number:
**PCT/KR2022/021602**

(87) International publication number:
**WO 2023/177056 (21.09.2023 Gazette 2023/38)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:   **15.03.2022   KR 20220031917**

(71) Applicants:
• **Samsung Life Public Welfare Foundation
  Seoul 04348 (KR)**
• **Research & Business Foundation
  SungKyunKwan University
  Gyeonggi-do 16419 (KR)**

• **Dongguk University Industry-Academic
  Cooperation Foundation
  Seoul 04620 (KR)**

(72) Inventors:
• **KIM, Hee Jin
  Seoul 06351 (KR)**
• **WON, Hong Hee
  Seoul 06351 (KR)**
• **KIM, Hang Rai
  Goyang-si, Gyeonggi-do 10326 (KR)**
• **JUNG, Sang Hyuk
  Seoul 06351 (KR)**

(74) Representative: **Ostertag & Partner Patentanwälte
mbB
Azenbergstraße 35
70174 Stuttgart (DE)**

(54)   **METHOD FOR PROVIDING INFORMATION FOR PREDICTING RISK GROUP FOR DEVELOPING ALZHEIMER?S DISEASE OR RISK GROUP FOR EARLY ONSET OF ALZHEIMER?S SYMPTOMS, OR RISK GROUP FOR DEVELOPING AMNESTIC MILD COGNITIVE IMPAIRMENT AND/OR PET-POSITIVE RISK GROUP FOR AMYLOID ? DEPOSITION, BASED ON EUROPEAN POPULATION DATA**

(57)   One aspect relates to a method for providing information for predicting a risk group for developing Alzheimer's disease dementia or a risk group for early onset of Alzheimer's symptoms, or a risk group for developing amnestic mild cognitive impairment and/or a positron emission tomography (PET)-positive risk group for amyloid β deposition, based on European population data. According to one aspect, the method makes it possible to accurately predict a risk group for developing Alzheimer's disease dementia or a risk group for early onset of Alzheimer's symptoms, or a risk group for developing amnestic mild cognitive impairment and/or a positron emission tomography (PET)-positive risk group for amyloid β deposition by using only at least 11 single-nucleotide polymorphisms, and the ability to predict the risk groups is further enhanced when up to and at most 39 additional single-nucleotide polymorphisms are used. In addition, the method further identifies age, sex, years of education, and APOE genotype as indicators, and thus the ability to predict the risk groups is further enhanced, and the risk groups can be predicted at an early stage with high accuracy.

EP 4 495 258 A1

[Fig 6a]

A. ADD Diagnosis

| APOE ε4 | Polygenic rist for ADD | No. of ADD / Total No. | | Adjusted OR (95% CI) | P value | P for trends |
|---|---|---|---|---|---|---|
| Non=carrier | - | 240/579 | | Reference | | |
| Carrier | - | 314/432 | | 3.16( 2.46=4.09) | <0.001 | |
| Non=carrier | Low PRS | 54/144 | | Re'ere"co | | <0.001 |
| | In:enned ate PRS | 62.147 | | 1.52(1.01-2.29) | 0.044 | |
| | High PRS | 64/148 | | 1.55(1.03-2.34) | 0.037 | |
| | Very high PRS | 60/139 | | 1.70(1.13-2.58) | 0.012 | |
| Carrier | Low PRS | 59/67 | | 2.63(1.63-4.29) | <0.001 | |
| | In:enned ate PRS | 78/107 | | 4.49(2.74-7.54) | <0.001 | |
| | High PRS | 85/113 | | 5.11(3.10-5.62) | <0.001 | |
| | Very high PRS | 92/115 | | 6.73(3.99-11.75) | <0.001 | |

0.0    1.0         5.0      10.0

**Description**

[Technical Field]

The present invention relates to a method for providing information for predicting a risk group for developing Alzheimer's disease dementia or a risk group for early onset of Alzheimer's symptoms, or a risk group for developing amnestic mild cognitive impairment and/or a PET-positive risk group for amyloid β deposition, based on European population data.

[Background Art]

Alzheimer's disease is the leading cause of dementia, affecting about 50 million people worldwide, and the number of patients with the disease is expected to triple by 2050 due to population aging. In particular, the disease is problematic in East Asia, where the population is aging rapidly. Almost a quarter of people with dementia are estimated to live in East Asia, and that number is expected to double every 20 years.

The pathological process of Alzheimer's disease begins long before clinical dementia develops. Therefore, it is very important for potential prevention and treatment strategies to identify individuals at high risk of developing Alzheimer's disease. Because the heritability of Alzheimer's disease is estimated to be 60 to 80%, genetic information may be used to identify individuals at high risk of developing Alzheimer's disease. Previous studies have demonstrated that polygenic risk scores (PRSs), which summarize the genetic effects of single nucleotide polymorphisms (SNPs) identified in genome-wide association studies (GWASs), can help distinguish individuals at high genetic risk for Alzheimer's disease.

However, previous genetic studies have been conducted primarily on European populations. Therefore, the generalizability of the PRS for non-European populations is not yet known. Recent studies have investigated the ability to predict European ancestry-derived PRS in non-European ancestry samples for various phenotypes. The PRS for Alzheimer's disease derived from European populations was evaluated in black and Caribbean Hispanic populations, but not in Hispanics. However, the performance of the PRS for Alzheimer's disease has not yet been evaluated in Asian populations.

Therefore, the present inventors have conducted studies in order to validate the possibility of inter-ethnic transfer of PRS for Alzheimer's disease in a Korean population using summarized statistics from a previous large-scale GWAS on a European population. The present inventors reproduced the next study results in an independent cohort of the Korean population, and assessed whether polygenic risk scores could be applied to predict Alzheimer's disease dementia (ADD), amnestic mild cognitive impairment (aMCI), or amyloid β (Aβ) deposition.

[Disclosure]

[Technical Problem]

The present invention is related to developing and providing a method for providing information for predicting a risk group for developing Alzheimer's disease or a risk group for early onset of Alzheimer's symptoms, or a risk group for developing amnestic mild cognitive impairment and a positron emission tomography (PET)-positive risk group for amyloid β deposition using a polygenic risk score (PRS) for Korean people, based on a method for predicting a risk group for Alzheimer's disease dementia using an existing polygenic risk score (PRS) for European people.

[Technical Solution]

One aspect of the present invention provides a method for providing information for predicting a risk group for developing Alzheimer's disease dementia (ADD) or a risk group for early onset of Alzheimer's symptoms, the method including: bringing a sample isolated from an individual in contact with a preparation capable of identifying the presence of risk alleles of a plurality of single-nucleotide polymorphisms (SNPs); and

determining the presence or absence of risk alleles of a plurality of single-nucleotide polymorphisms in the sample, wherein the plurality of single-nucleotide polymorphisms includes rs6733839, rs1582763, rs679515, rs1532276, rs3851179, rs1752684, rs56201148, rs67472071, rs35832505, rs12151021, and rs73223431.

The method for providing information for predicting a risk group for developing Alzheimer's disease dementia (ADD) or a risk group for early onset of Alzheimer's symptoms was derived by using European-based meta-GWAS results obtained from an inverse variance-weighted fixed-effect meta-analysis of various European GWAS results and analyzing a predictive performance by including single-nucleotide polymorphisms in a P-value threshold range of the GWAS.

According to one aspect, the method may predict a risk group for developing Alzheimer's disease dementia or a risk group for early onset of Alzheimer's symptoms with excellent accuracy by confirming the presence or absence of risk alleles of at least 11 single-nucleotide polymorphisms in a sample.

The term "individual" may refer to a mammal, for example, a mouse, rat, cat, guinea pig, hamster, dog, monkey, chimpanzee, human, and the like, and specifically a human.

The sample may be blood, plasma, serum, tissue, cells, lymphatic fluid, bone marrow fluid, saliva, ocular fluid, semen, brain extract, spinal fluid, synovial fluid, thymic fluid, ascitic fluid, amniotic fluid, cell tissue fluid, and cell culture fluid, and may be specifically blood, plasma, serum, tissue, cells, lymphatic fluid, bone marrow fluid, cell tissue fluid, and cell culture fluid, and more specifically blood or saliva.

The preparation may be selected from the group consisting of a primer, a probe, an aptamer, an antibody, a peptide, and combinations thereof, capable of specifically binding to a base sequence including the single-nucleotide polymorphism or a protein encoded by the base sequence.

The term "primer" refers to a nucleic acid sequence that can form complementary base pairs with a template strand and serve as a starting point for template strand copying, and may be, for example, a nucleic acid sequence of 5 to 50 amino acids. The primer is usually synthesized, but may also be used on naturally occurring nucleic acids. The sequence of the primer does not necessarily have to be exactly the same as the sequence of the template, but it must be sufficiently complementary to hybridize with the template.

The term "probe" refers to a material capable of specifically binding to a target material to be detected in a sample, and refers to a material in a sample of which the presence of a target material can be specifically confirmed through the binding.

The term "aptamer" refers to a small single-stranded nucleic acid (DNA or RNA) fragment that has the characteristics of being able to bind with high affinity and specificity to various types of substances, from low molecular weight compounds to proteins, and may be, for example, a single-stranded nucleic acid fragment consisting of 10 to 60 nucleotides.

The term "antibody" refers to a substance that specifically binds to an antigen to cause an antigen-antibody reaction, and may be a chimeric antibody, a humanized antibody, a human antibody, a synthetic antibody and/or an affinity matured antibody.

The term "peptide" refers to a polymer consisting of two or more amino acids linked together through amide bonds (or peptide bonds).

The term "single-nucleotide polymorphisms (SNPs)" refers to the presence of two or more alleles at a single gene locus, where only a single nucleotide is different at a polymorphic site.

The term "Alzheimer's disease dementia" refers to the dementia symptoms induced by Alzheimer's disease. Alzheimer's disease described above is the most common degenerative brain disease that causes dementia, which was first reported by Dr. Alzheimer in Germany, and it is known that as Alzheimer's disease progresses, overall cognitive functions including memory gradually weaken.

The term "onset" refers to the beginning of a disease, and the term "early onset of symptoms" refers to the manifestation of various states or forms appearing when one is suffering from a disease at the early stage of the onset of the disease.

The determining of the presence or absence of risk alleles of a plurality of single-nucleotide polymorphisms in the sample may include determining the presence or absence of risk alleles of the plurality of single-nucleotide polymorphisms by detecting a nucleic acid (for example, DNA, RNA, and the like) and/or a protein in the sample to analyze a genotype. Specifically, the genotype may be analyzed by detecting DNA in a sample using, for example, an Illumina Asian Screening Array Bead Chip (ASA chip, CA).

In one aspect, the plurality of single-nucleotide polymorphisms may further include one or more single-nucleotide polymorphisms selected from the group consisting of rs12358692, rs11218343, rs6014724, rs6805148, rs17125924, rs35695568, rs11767557, rs3795065, rs3752786, rs11623019, rs12590654, rs11039165, rs28482811, rs3135348, rs9381563, rs9268112, rs3865444, rs11230227, rs9271375, rs2293579, rs7831810, rs11168036, rs598561, rs3017432, rs2526378, rs8111708, rs7805776, and rs12197146.

When the plurality of single-nucleotide polymorphisms further include one or more single-nucleotide polymorphisms as described above, the method has better performance when predicting a risk group for developing Alzheimer's disease dementia or a risk group for early onset of Alzheimer's symptoms, and the higher the number of single-nucleotide polymorphisms that are further included, the better the predictive performance of the method may be.

In one aspect, the method may further include obtaining a score for a single-nucleotide polymorphism by assigning a score of 1 to a single-nucleotide polymorphism determined to indicate the presence of a risk allele in the sample among the plurality of single-nucleotide polymorphisms, wherein among the plurality of single-nucleotide polymorphisms, a single-nucleotide polymorphism determined to be absent from the sample is assigned a score of 0.

In one aspect, the method may further include obtaining a first polygenic risk score (PRS) value by multiplying the assigned score for the single-nucleotide polymorphism by a coefficient (β) assigned for each of the following single-nucleotide polymorphisms, and adding all the multiplied values, and

the coefficient of rs6733839 may be 0.1693, the coefficient of rs1582763 may be -0.1232, the coefficient of rs679515 may be 0.1508, the coefficient of rs1532276 may be -0.1266, the coefficient of rs3851179 may be -0.1198, the coefficient of rs1752684 may be 0.1432, the coefficient of rs56201148 may be -0.1137, the coefficient of rs67472071 may be -0.0981, the coefficient of rs35832505 may be 0.1213, the coefficient of rs12151021 may be 0.1071, the coefficient of rs73223431 may be 0.0936, the coefficient of rs12358692 may be 0.6429, the coefficient of rs11218343 may be -0.2053, the coefficient of rs6014724 may be -0.1319, the coefficient of rs17125924 may be 0.1222, the coefficient of rs35695568 may be 0.1152, the coefficient of rs11767557 may be -0.1028, the coefficient of rs3795065 may be 0.0968, the coefficient of rs3752786 may be 0.0964, the coefficient of rs11623019 may be 0.0913, the coefficient of rs12590654 may be -0.0906, the coefficient of rs11039165 may be 0.0894, the coefficient of rs28482811 may be -0.0872, the coefficient of rs3135348 may be 0.0837, the coefficient of rs9381563 may be 0.0821, the coefficient of rs9268112 may be 0.0815, the coefficient of rs3865444 may be -0.0804, the coefficient rs11230227 may be 0.0792, the coefficient of rs9271375 may be -0.0789, the coefficient of rs2293579 may be 0.0771, the coefficient of rs7831810 may be -0.0765, the coefficient of rs11168036 may be 0.0754, the coefficient of rs598561 may be 0.0747, the coefficient of rs3017432 may be -0.0735, the coefficient of rs2526378 may be 0.0717, the coefficient of rs8111708 may be 0.0696, the coefficient of rs7805776 may be -0.0695, and the coefficient of rs12197146 may be -0.0674.

The term "polygenic risk score (PRS)" refers to a score that predicts the risk of developing a disease by evaluating various genetic factors associated with one disease.

The term "gene" refers to a segment of DNA involved in producing a polypeptide chain. The DNA segment may include intervening sequences (introns) between individual coding segments (exons), as well as the regions preceding and following the coding region (leader or trailer) involved in the transcription/translation of a gene product and the regulation of transcription/translation.

In one aspect, the method may further include determining that when the first PRS value is higher than the first PRS value of an individual not having Alzheimer's disease dementia, the individual is in a high risk group for developing Alzheimer's disease dementia or in a high risk group for early onset of Alzheimer's symptoms.

In one aspect, when the plurality of single-nucleotide polymorphisms further include rs12358692, rs11218343, rs6014724, rs6805148, rs17125924, rs35695568, rs11767557, rs3795065, rs3752786, rs11623019, rs12590654, rs11039165, rs28482811, rs3135348, rs9381563, rs9268112, rs3865444, rs11230227, rs9271375, rs2293579, rs7831810, rs11168036, rs598561, rs3017432, rs2526378, rs8111708, rs7805776, and rs12197146, the obtaining of the first PRS value may include calculating, for example, the first PRS value using the following Mathematical Formula 1:

the first PRS value (39 SNPs) = rs12358692*0.6429 + rs11218343*-0.2053 + rs6733839*0.1693 + rs679515*0.1508 + rs1752684*0.1432 + rs6014724*-0.1319 + rs6805148*-0.1293 + rs1532276*-0.1266 + rs1582763*-0.1232 + rs17125924*0.1222 + rs35832505*0.1213 + rs3851179*-0.1198 + rs35695568*0.1152 + rs56201148*-0.1137 + rs12151021*0.1071 + rs11767557*-0.1028 + rs67472071*-0.0981 + rs3795065*0.0968 + rs3752786*0.0964 + rs73223431*0.0936 + rs11623019*0.0913 + [Mathematical Formula 1] rs12590654*-0.0906 + rs11039165*0.0894 + rs28482811*-0.0872 + rs3135348*0.0837 + rs9381563*0.0821 + rs9268112*0.0815 + rs3865444*-0.0804 + rs11230227*0.0792 + rs9271375*-0.0789 + rs2293579*0.0771 + rs7831810*-0.0765 + rs11168036*0.0754 + rs598561*0.0747 + rs3017432*-0.0735 + rs2526378*0.0717 + rs8111708*0.0696 + rs7805776*-0.0695 + rs12197146*-0.0674.

In one aspect, the method may further include identifying one or more indicators selected from the group consisting of the individual's age, sex, years of education, and APOE genotype.

In one aspect, when the method further includes identifying one or more indicators selected from the group consisting of the individual's age, sex, years of education, and APOE genotype, the method has better performance when predicting a risk group for developing Alzheimer's disease dementia or a risk group for early onset of Alzheimer's symptoms, and the higher the number of indicators identified, the better the predictive performance of the method may be.

The term "Apolipoprotein (APOE) genotype" refers to the genotype of a gene encoding Apolipoprotein E. APOE is a gene encoding the constituent proteins of lipoproteins, which are normal components of plasma such as high density lipoprotein (HDL), low density lipoprotein (LDL), and very low density lipoprotein (VLDL), and is located on chromosome 19 and plays a key role in regulating fat metabolism after damage to the central and peripheral nervous systems as a fat transporter. The APOE gene has three allelic genotypes: $\varepsilon2$, $\varepsilon3$, and $\varepsilon4$, and is classified into a total of six genotypes: $\varepsilon2/\varepsilon2$, $\varepsilon3/3$, $\varepsilon4/\varepsilon4$, $\varepsilon2/\varepsilon3$, $\varepsilon2/\varepsilon4$, and $\varepsilon3/\varepsilon4$ because every person inherits one allele of the APOE gene from each parent. Among the allelic genotypes, $\varepsilon2$ and $\varepsilon4$ are known to be associated with Alzheimer's disease dementia, with the $\varepsilon2$ type known to decrease the risk of Alzheimer's disease dementia and the $\varepsilon4$ type known to increase the risk of Alzheimer's disease dementia.

In one aspect, the method may further include obtaining a score for each indicator by assigning a score (natural number) based on the number of years of education in the case of the age and years of education among the indicators of the individual,

assigning a score of 1 for males and a score of 2 for females in the case of sex among the indicators of the individual, and

assigning a score of 0 for ε2/ε2, ε2/ε3, and ε3/ε3 and a score of 1 for ε2/ε4, ε3/ε4, and ε4/ε4 in the case of APOE genotype among the indicators of the individual.

When the indicator is age in the obtaining of the score for each of the indicators, a score (natural number) is assigned based on the number of years, and for example, when the age is 72 years and 6 months, a score of 72 may be assigned.

When the indicator is years of education in the obtaining of the score for each of the indicators, a score (natural number) is assigned based on the number of years, and for example, when the years of education are 11 years and 2 months, a score of 11 may be assigned.

When the indicator is sex in the obtaining of the score for each of the indicators, if gender, a score of 1 and a score of 2 may be assigned for males and females, respectively.

The method may further include obtaining a score for each indicator by assigning a score of 0 for ε2/ε2, ε2/ε3, and ε3/ε3 and a score of 1 for ε2/ε4, ε3/ε4, and ε4/ε4 in the case of APOE genotype among the indicators of the individual.

In one aspect, the method may further include obtaining a second PRS value by multiplying the assigned score for each indicator by a coefficient (β) assigned for each of the following indicators, and adding the first PRS value and a coefficient (β) assigned for the following first PRS value to the multiplied values, and

the coefficient of the age may be 0.02879, the coefficient of the sex may be 0.03618, the coefficient of the years of education may be -0.02615, the coefficient of the APOE genotype may be 1.3712, and the coefficient of the first PRS value may be 0.66119.

The obtaining of the second PRS value may calculate, for example, the second PRS value using a mathematical formula represented by the following Mathematical Formula 2:

$$\text{Second PRS value (including 4 factors)} = \text{first PRS} *0.6611\ 9 + \text{age}*0.02879 + \text{sex}*0.03618 + \text{years of education}*-0.02615 + \text{APOE}\varepsilon4*1.37120. \qquad \text{[Mathematical Formula 2]}$$

In one aspect, the method may further include determining that when the second PRS value is higher than the second PRS value of an individual not having Alzheimer's disease dementia, the individual is in a high risk group for developing Alzheimer's disease dementia or in a high risk group for early onset of Alzheimer's symptoms.

Another aspect of the present invention provides a method for providing information for predicting a risk group for developing Alzheimer's disease dementia or a risk group for early onset of Alzheimer's symptoms, the method including: bringing a sample isolated from an individual in contact with a preparation capable of identifying the presence of risk alleles of a plurality of single-nucleotide polymorphisms (SNPs); and

determining the presence or absence of risk alleles of the plurality of single-nucleotide polymorphisms, wherein the plurality of single-nucleotide polymorphisms include rs6733839, rs1582763, rs679515, rs1532276, rs3851179, rs1752684, rs56201148, rs67472071, rs35832505, rs12151021, rs12358692, rs11218343, rs6014724, rs6805148, rs17125924, rs35695568, rs11767557, rs3752786, rs11623019, rs12590654, rs28482811, rs9381563, rs11230227, rs9271375, rs7831810, rs11168036, rs598561, rs3017432, rs8111708, rs7805776, and rs12197146.

The "individual," "sample," "single-nucleotide polymorphism," "preparation," "Alzheimer's disease dementia," and the like may be within the above-described scope.

The method for providing information for predicting a risk group for developing Alzheimer's disease dementia (ADD) or a risk group for early onset of Alzheimer's symptoms was derived by using European-based meta-GWAS results obtained from an inverse variance-weighted fixed-effect meta-analysis of various European GWAS results and analyzing predictive performance by including single-nucleotide polymorphisms in a P-value threshold range of the GWAS.

According to one aspect, the method may predict a risk group for developing Alzheimer's disease dementia or a risk group for early onset of Alzheimer's symptoms with excellent accuracy by confirming the presence or absence of risk alleles of 31 single-nucleotide polymorphisms in a sample.

Still another aspect of the present invention provides a composition for predicting a risk group for developing Alzheimer's disease dementia or a risk group for early onset of Alzheimer's symptoms, including a preparation capable of confirming the presence or absence of risk alleles of a plurality of single-nucleotide polymorphisms (SNPs) in a sample isolated from an individual,

wherein the plurality of single-nucleotide polymorphisms are rs6733839, rs1582763, rs679515, rs1532276, rs3851179, rs1752684, rs56201148, rs67472071, rs35832505, rs12151021, and rs73223431.

The "individual," "sample," "single-nucleotide polymorphism," "Alzheimer's disease dementia," and the like may be within the above-described scope.

The composition for predicting a risk group for developing Alzheimer's disease dementia (ADD) or a risk group for early onset of Alzheimer's symptoms was invented by using European-based meta-GWAS results obtained from an inverse variance-weighted fixed-effect meta-analysis of various European GWAS results and analyzing a predictive performance by including single-nucleotide polymorphisms in a P-value threshold range of the GWAS.

According to one aspect, the composition may predict a risk group for developing Alzheimer's disease dementia or a risk group for early onset of Alzheimer's symptoms with excellent accuracy by confirming the presence or absence of risk alleles of at least 11 single-nucleotide polymorphisms in a sample.

The preparation may be selected from the group consisting of a primer, a probe, an aptamer, an antibody, a peptide, and combinations thereof, capable of specifically binding to a base sequence including the single-nucleotide polymorphism or a protein encoded by the base sequence.

In one aspect, the plurality of single-nucleotide polymorphisms may further include one or more single-nucleotide polymorphisms selected from the group consisting of rs12358692, rs11218343, rs6014724, rs6805148, rs17125924, rs35695568, rs11767557, rs3795065, rs3752786, rs11623019, rs12590654, rs11039165, rs28482811, rs3135348, rs9381563, rs9268112, rs3865444, rs11230227, rs9271375, rs2293579, rs7831810, rs11168036, rs598561, rs3017432, rs2526378, rs8111708, rs7805776, and rs12197146.

When the plurality of single-nucleotide polymorphisms further include one or more single-nucleotide polymorphisms as described above, the composition has better performance when predicting a risk group for developing Alzheimer's disease dementia or a risk group for early onset of Alzheimer's symptoms, and the higher the number of single-nucleotide polymorphisms is, the better the predictive performance of the composition may be.

In one aspect, the composition may further include a preparation capable of identifying the APOE genotype of the individual in the sample.

In one aspect, when the composition further includes a preparation capable of identifying the APOE genotype of the individual in the sample, the composition has better performance when predicting a risk group for developing Alzheimer's disease dementia or a risk group for early onset of Alzheimer's symptoms.

Yet another aspect of the present invention provides a kit for predicting a risk group for developing Alzheimer's disease dementia or a risk group for early onset of Alzheimer's symptoms, including the composition.

The "Alzheimer's disease dementia" may be within the above-described scope.

The kit may be a reverse transcription polymerase chain reaction (RT-PCR) kit or a DNA chip kit.

The kit for predicting a risk group for developing Alzheimer's disease dementia or a risk group for early onset of Alzheimer's symptoms may additionally include one or more other constituent compositions, solutions or devices suitable for an analytical method.

As an example, the kit may be a diagnostic kit characterized by including essential elements required for carrying out a reverse transcription polymerase reaction, and in addition to a primer capable of specifically binding to the APOE gene or a base sequence including the single-nucleotide polymorphisms, the kit may include a test tube or another suitable container, a reaction buffer (pH and magnesium concentration are various), deoxynucleotides (dNTPs), an enzyme such as Taq-polymerase and a reverse transcriptase, DNAse, RNAse inhibitor DEPC-water, sterile water, and the like.

As another example, the kit may be a diagnostic kit characterized by including essential elements required for carrying out a DNA chip. The DNA chip kit may include a substrate to which cDNA or oligonucleotides corresponding to genes or fragments thereof are attached, as well as a reagent, a preparation, an enzyme, and the like for producing a fluorescently labeled probe. Further, the substrate may include a cDNA or oligonucleotide corresponding to a control gene or a fragment thereof.

According to one aspect, the kit may predict a risk group for developing Alzheimer's disease dementia or a risk group for early onset of Alzheimer's symptoms with excellent accuracy by confirming the presence or absence of risk alleles of at least 11 single-nucleotide polymorphisms in a sample.

Yet another aspect of the present invention provides a method for providing information for predicting a risk group for developing amnestic mild cognitive impairment, the method including: bringing a sample isolated from an individual in contact with a preparation capable of identifying the presence of risk alleles of a plurality of single-nucleotide polymorphisms (SNPs); and

determining the presence or absence of risk alleles of the plurality of single-nucleotide polymorphisms, wherein the plurality of single-nucleotide polymorphisms include rs6733839, rs1582763, rs679515, rs1532276, rs3851179, rs1752684, rs56201148, rs67472071, rs35832505, rs12151021, and rs73223431.

The "individual," "sample," "single-nucleotide polymorphism," "preparation," and the like may be within the above-described scope.

The term "amnestic mild cognitive impairment" refers to a type of mild cognitive impairment (MCI) in which memory is primarily affected, and "mild cognitive impairment" refers to a type of mental deterioration with a clinical dementia rating (CDR) of less than 1.

The method for providing information for predicting a risk group for developing amnestic mild cognitive impairment was derived by using European-based meta-GWAS results obtained from an inverse variance-weighted fixed-effect meta-analysis of various European GWAS results and analyzing a predictive performance by including single-nucleotide polymorphisms in a P-value threshold range of the GWAS.

According to one aspect, the method may predict a risk group for developing amnestic mild cognitive impairment with excellent accuracy by confirming the presence or absence of risk alleles of at least 11 single-nucleotide polymorphisms in a sample.

In one aspect, the plurality of single-nucleotide polymorphisms may further include one or more single-nucleotide polymorphisms selected from the group consisting of rs12358692, rs11218343, rs6014724, rs6805148, rs17125924, rs35695568, rs11767557, rs3795065, rs3752786, rs11623019, rs12590654, rs11039165, rs28482811, rs3135348, rs9381563, rs9268112, rs3865444, rs11230227, rs9271375, rs2293579, rs7831810, rs11168036, rs598561, rs3017432, rs2526378, rs8111708, rs7805776, and rs12197146.

When the plurality of single-nucleotide polymorphisms further include one or more single-nucleotide polymorphisms as described above, the method has better performance when predicting a risk group for developing amnestic mild cognitive impairment, and the higher the number of single-nucleotide polymorphisms that are further included, the better the predictive performance of the method may be.

In one aspect, the method may further include identifying one or more indicators selected from the group consisting of the individual's age, sex, years of education, and APOE genotype.

In one aspect, when the method further includes identifying one or more indicators selected from the group consisting of the individual's age, sex, years of education, and APOE genotype, the method has better performance when predicting a risk group for developing amnestic mild cognitive impairment, and the higher the number of indicators identified, the better the predictive performance of the method may be.

Yet another aspect of the present invention provides a composition for predicting a risk group for developing amnestic mild cognitive impairment, including a preparation capable of confirming the presence or absence of risk alleles of a plurality of single-nucleotide polymorphisms (SNPs) in a sample isolated from an individual, wherein the plurality of SNPs includes rs6733839, rs1582763, rs679515, rs1532276, rs3851179, rs1752684, rs56201148, rs67472071, rs35832505, rs12151021, and rs73223431.

The "individual," "sample," "single-nucleotide polymorphism," "amnestic mild cognitive impairment," and the like may be within the above-described scope.

The composition for predicting a risk group for developing amnestic mild cognitive impairment was invented by using European-based meta-GWAS results obtained from an inverse variance-weighted fixed-effect meta-analysis of various European GWAS results and analyzing predictive performance by including single-nucleotide polymorphisms in a P-value threshold range of the GWAS.

According to one aspect, the composition may predict a risk group for developing amnestic mild cognitive impairment with excellent accuracy by confirming the presence or absence of risk alleles of at least 11 single-nucleotide polymorphisms in a sample.

The preparation may be selected from the group consisting of a primer, a probe, an aptamer, an antibody, a peptide, and combinations thereof, capable of specifically binding to a base sequence including the single-nucleotide polymorphism or a protein encoded by the base sequence.

In one aspect, the plurality of single-nucleotide polymorphisms may further include one or more single-nucleotide polymorphisms selected from the group consisting of rs12358692, rs11218343, rs6014724, rs6805148, rs17125924, rs35695568, rs11767557, rs3795065, rs3752786, rs11623019, rs12590654, rs11039165, rs28482811, rs3135348, rs9381563, rs9268112, rs3865444, rs11230227, rs9271375, rs2293579, rs7831810, rs11168036, rs598561, rs3017432, rs2526378, rs8111708, rs7805776, and rs12197146.

When the plurality of single-nucleotide polymorphisms further include one or more single-nucleotide polymorphisms as described above, the composition has better performance when predicting a risk group for developing amnestic mild cognitive impairment, and the higher the number of single-nucleotide polymorphisms that are further included, the better the predictive performance of the composition may be.

In one aspect, the composition may further include a preparation capable of identifying the APOE genotype of the individual in the sample.

In one aspect, when the composition further includes a preparation capable of identifying the APOE genotype of the individual in the sample, the composition has better performance when predicting a risk group for developing amnestic mild cognitive impairment.

Yet another aspect of the present invention provides a kit for predicting a risk group for developing amnestic mild cognitive impairment, including the composition.

The "amnestic mild cognitive impairment" and "kit" may be within the above-described scope.

According to one aspect, the kit may predict a risk group for developing amnestic mild cognitive impairment with excellent accuracy by confirming the presence or absence of risk alleles of at least 11 single-nucleotide polymorphisms in a sample.

Yet another aspect of the present invention provides a method for providing information for predicting a positron emission tomography (PET)-positive risk group for amyloid β deposition, the method including: bringing a sample isolated from an individual in contact with a preparation capable of identifying the presence of risk alleles of a plurality of single-nucleotide polymorphisms (SNPs); and

> determining the presence or absence of risk alleles of the plurality of single-nucleotide polymorphisms,
> wherein the plurality of single-nucleotide polymorphisms include rs6733839, rs1582763, rs679515, rs1532276, rs3851179, rs1752684, rs56201148, rs67472071, rs35832505, rs12151021, and rs73223431.

The "individual," "sample," "single-nucleotide polymorphism," "preparation," and the like may be within the above-described scope.

The term "positron emission tomography (PET)-positive risk group for amyloid β deposition" refers to visual confirmation of the presence or absence of amyloid-β deposition through amyloid positron emission tomography (PET).

The term "positron emission tomography (PET)" refers to a technology in which a radiopharmaceutical which emits positrons is injected intravenously or inhaled into the body, and then the gamma rays generated by a positron annihilation phenomenon are measured by a circular ring-shaped detector that surrounds the body as they pass through the body, and the distribution of positron-emitting nuclides within the body is processed by a computer to reconstruct an image.

The method for providing information for predicting a PET-positive risk group for amyloid β deposition was derived by using European-based meta-GWAS results obtained from an inverse variance-weighted fixed-effect meta-analysis of various European GWAS results and analyzing predictive performance by including single-nucleotide polymorphisms in a P-value threshold range of the GWAS.

According to one aspect, the method may predict a PET-positive risk group for amyloid β deposition with excellent accuracy by confirming the presence or absence of risk alleles of at least 11 single-nucleotide polymorphisms in a sample.

In one aspect, the plurality of single-nucleotide polymorphisms may further include one or more single-nucleotide polymorphisms selected from the group consisting of rs12358692, rs11218343, rs6014724, rs6805148, rs17125924, rs35695568, rs11767557, rs3795065, rs3752786, rs11623019, rs12590654, rs11039165, rs28482811, rs3135348, rs9381563, rs9268112, rs3865444, rs11230227, rs9271375, rs2293579, rs7831810, rs11168036, rs598561, rs3017432, rs2526378, rs8111708, rs7805776, and rs12197146.

When the plurality of single-nucleotide polymorphisms further include one or more single-nucleotide polymorphisms as described above, the method has better performance when predicting a PET-positive risk group for amyloid β deposition, and the higher the number of single-nucleotide polymorphisms that are further included, the better the predictive performance of the method may be.

In one aspect, the method may further include identifying one or more indicators selected from the group consisting of the individual's age, sex, years of education, and APOE genotype.

In one aspect, when the method further includes identifying one or more indicators selected from the group consisting of the individual's age, sex, years of education, and APOE genotype, the method has better performance when predicting a PET-positive risk group for amyloid β deposition, and the higher the number of indicators identified, the better the predictive performance of the method may be.

Yet another aspect of the present invention provides a composition for predicting a PET-positive risk group for amyloid β deposition, including a preparation capable of confirming the presence or absence of risk alleles of a plurality of single-nucleotide polymorphisms (SNPs) in a sample isolated from an individual, wherein the plurality of single-nucleotide polymorphisms are rs6733839, rs1582763, rs679515, rs1532276, rs3851179, rs1752684, rs56201148, rs67472071, rs35832505, rs12151021, and rs73223431.

The "individual," "sample," "single-nucleotide polymorphism," "positron emission tomography (PET)-positive risk group for amyloid β deposition," and the like may be within the above-described scope.

The composition for predicting a PET-positive risk group for amyloid β deposition was invented by using European-based meta-GWAS results obtained from an inverse variance-weighted fixed-effect meta-analysis of various European GWAS results and analyzing predictive performance by including single-nucleotide polymorphisms in a P-value threshold range of the GWAS.

According to one aspect, the composition may predict a PET-positive risk group for amyloid β deposition with excellent accuracy by confirming the presence or absence of risk alleles of at least 11 single-nucleotide polymorphisms in a sample.

The preparation may be selected from the group consisting of a primer, a probe, an aptamer, an antibody, a peptide, and combinations thereof, capable of specifically binding to a base sequence including the single-nucleotide polymorphism or a protein encoded by the base sequence.

In one aspect, the plurality of single-nucleotide polymorphisms may further include one or more single-nucleotide polymorphisms selected from the group consisting of rs12358692, rs11218343, rs6014724, rs6805148, rs17125924, rs35695568, rs11767557, rs3795065, rs3752786, rs11623019, rs12590654, rs11039165, rs28482811, rs3135348, rs9381563, rs9268112, rs3865444, rs11230227, rs9271375, rs2293579, rs7831810, rs11168036, rs598561, rs3017432, rs2526378, rs8111708, rs7805776, and rs12197146.

When the plurality of single-nucleotide polymorphisms further include one or more single-nucleotide polymorphisms as described above, the composition has better performance when predicting a PET-positive risk group for amyloid β deposition, and the higher the number of single-nucleotide polymorphisms that are further included, the better the predictive performance of the composition may be.

In one aspect, the composition may further include a preparation capable of identifying the APOE genotype of the individual in the sample.

In one aspect, when the composition further includes a preparation capable of identifying the APOE genotype of the individual in the sample, the composition has better performance when predicting a PET-positive risk group for amyloid β deposition.

Yet another aspect of the present invention provides a kit for predicting a PET-positive risk group for amyloid β deposition, including the composition.

The "positron emission tomography (PET)-positive risk group for amyloid β deposition" and "kit" may be within the above-described scope.

According to one aspect, the kit may predict a PET-positive risk group for amyloid β deposition with excellent accuracy by confirming the presence or absence of risk alleles of at least 11 single-nucleotide polymorphisms in a sample.

Yet another aspect of the present invention provides a use of a preparation capable of confirming the presence or absence of risk alleles of a plurality of single-nucleotide polymorphisms (SNPs) in a sample isolated from an individual for predicting a risk group for developing Alzheimer's disease dementia or a risk group for early onset of Alzheimer's symptoms,

wherein the plurality of single-nucleotide polymorphisms are rs6733839, rs1582763, rs679515, rs1532276, rs3851179, rs1752684, rs56201148, rs67472071, rs35832505, rs12151021, and rs73223431.

The "individual," "sample," "single-nucleotide polymorphism," "Alzheimer's disease dementia," and the like may be within the above-described scope.

Yet another aspect of the present invention provides a use of a preparation capable of confirming the presence or absence of risk alleles of a plurality of single-nucleotide polymorphisms (SNPs) in a sample isolated from an individual for predicting a risk group for developing amnestic mild cognitive impairment,

wherein the plurality of single-nucleotide polymorphisms are rs6733839, rs1582763, rs679515, rs1532276, rs3851179, rs1752684, rs56201148, rs67472071, rs35832505, rs12151021, and rs73223431.

The "individual," "sample," "single-nucleotide polymorphism," "amnestic mild cognitive impairment," and the like may be within the above-described scope.

Yet another aspect of the present invention provides a use of a preparation capable of confirming the presence or absence of risk alleles of a plurality of single-nucleotide polymorphisms (SNPs) in a sample isolated from an individual for predicting a PET-positive risk group for amyloid β deposition,

wherein the plurality of single-nucleotide polymorphisms are rs6733839, rs1582763, rs679515, rs1532276, rs3851179, rs1752684, rs56201148, rs67472071, rs35832505, rs12151021, and rs73223431.

The "individual," "sample," "single-nucleotide polymorphism," "positron emission tomography (PET)-positive risk group for amyloid β deposition," and the like may be within the above-described scope.

[Advantageous Effects]

According to one aspect, the method makes it possible to accurately predict a risk group for developing Alzheimer's disease dementia or a risk group for early onset of Alzheimer's symptoms, or a risk group for developing amnestic mild cognitive impairment and/or a positron emission tomography (PET)-positive risk group for amyloid β deposition by using only at least 11 single-nucleotide polymorphisms, and the ability to predict the risk groups is further enhanced when up to and at most 39 additional single-nucleotide polymorphisms are used. In addition, the method further identifies age, sex, years of education, and APOE genotype as indicators, and thus the ability to predict the risk groups is further enhanced, and the risk groups can be predicted at an early stage with high accuracy.

[Description of Drawings]

FIGS. 1A and 1B are views illustrating the results of a comparison of principal components between Korean study populations and genome project populations.

FIG. 2 is a set of views illustrating the results of PRS distribution among study subjects according to genotype array.

FIG. 3 is a view illustrating an overview of the study dataset and analysis steps.

FIG. 4 is a set of views illustrating the results of distribution for Nagelkerke $R^2$ values of each population-based PRS across the single-nucleotide polymorphism selection threshold.

FIG. 5 is a view illustrating the scatter plots of beta coefficients for 39 single-nucleotide polymorphisms.

FIGS. 6A to 6C are views illustrating the results of Forest plots of Alzheimer's Disease Dementia (ADD)-related outcomes by PRS group and APOE ε4 status.

[Modes of the Invention]

Hereinafter, the present invention will be described in more detail through examples. However, these examples are provided only for exemplarily describing the present invention, and the scope of the present invention is not limited by these examples.

**Examples**

**1. Validation and amnestic mild cognitive impairment (aMCI) dataset**

From January 2013 to July 2019, 1,255 Korean subjects were recruited from 14 hospitals in the Republic of Korea. Specifically, 954 participants were recruited from Samsung Seoul Hospital, 202 from the Korean Brain Aging Study for Early Diagnosis and Prediction of AD, and 99 from a multicenter clinical research platform study based on the Dementia Cohort (Table 1). Based on detailed neuropsychological test results, subjects diagnosed with Alzheimer's disease dementia (ADD) or amnestic mild cognitive impairment (aMCI) or cognitively unimpaired (CU) were included, and the diagnosis of subjects at the most recent assessment point was used. Alzheimer's disease dementia (ADD) was defined by the core clinical criteria for Alzheimer's disease dementia (ADD) according to the National Institute on Aging-Alzheimer's Association. Amnestic mild cognitive impairment (aMCI) was defined according to the following criteria modified from Peterson's criteria: (i) normal activities of daily living performance, (ii) objective memory impairment, that is, performance ability below the 16th percentile of age- and education-matching norms in verbal or visual memory tests, and (iii) no dementia.

Subj ects were excluded when they had (i) causative gene mutations for Alzheimer's disease (AD) in known genes such as Presenilin-1 (PSEN1), Presenilin-2 (PSEN2) and amyloid-beta precursor protein (APP), (ii) structural abnormalities found by brain magnetic resonance imaging such as severe cerebral ischemia, cerebral infarction or brain tumors, or (iii) other medical or psychiatric diseases that may induce cognitive decline. All subjects were provided written informed consent, and the study was approved by the Institutional Review Board at each center.

[Table 1]

| Characteristics | Validation dataset (n=1,033) | | Replication dataset (n=379) | | aMCI dataset |
|---|---|---|---|---|---|
| | CU (n=479) | ADD (n=554) | CU (n=220) | ADD (n=159) | aMCI (n=222) |
| Age, mean (SD), year | 70.7±7.6 | 73.1±10.0 | 67.8±9.2 | 72.6±8.6 | 73.0±8.2 |
| Female sex, no. (%) | 282 (58.9) | 348 (62.8) | 139 (63.2%) | 91 (57.2%) | 109 (49.1) |
| Education, mean (SD), year | 11.2±4.9 | 10.4±5.0 | 11.3±4.6 | 9.7±5.3 | 11.9±4.7 |
| *APOE* ε4 carrier, no. (%) | 118 (24.6%) | 314 (56.7%) | 55 (25.0%) | 74 (46.5%) | 79 (35.6%) |
| Amyloid PET positivity, no. (%) | 63 (14.0%) | 479 (87.7%) | 33 (15.0%) | 119 (74.8%) | 108 (49.3%) |

Abbreviations: CU, cognitively unimpaired; aMCI, amnestic mild cognitive impairment; ADD, Alzheimer's disease dementia; SD, standard deviation; and PET, positron emission tomography.

### 2. Replication dataset

For a replication dataset, data from 379 Korean subjects was secured from 20 referral hospitals in Korea. Specifically, data on 125 subjects recruited from the biobank of the Chronic Cerebrovascular Disease Consortium from 2016 to 2018 was secured, and data on the remaining 254 subjects was secured from the PRECISION medicine platform for mild cognitive impairment based on Multi-omics, imaging, and Evidence-based R&BD (PREMIERM) cohort. Further, Alzheimer's disease dementia (ADD) or cognitively unimpaired (CU) was included according to the same criteria of the validation dataset.

### 3. Genotype analysis and statistical imputation

DNA specimens were genotyped using Illumina Asian Screening Array BeadChips (ASA chips, CA, USA). A portion of the specimens (n = 125) was genotyped using an Affymetrix custom-made Korea Biobank Array chip (KBA chip, Affymetrix, CA, USA), and quality control (QC) was performed on the data for two types of single-nucleotide polymorphisms (SNPs). SNPs were removed according to the following criteria: (i) call rate <98%, (ii) minor allele frequency (MAF) <1%, or (iii) genotype frequency that deviates significantly from Hardy-Weinberg equilibrium with a P-value of $<10^{-6}$. After quality control (QC), the genotype data was directly subjected to genotype estimation for mutations for which no genotype was assigned, and statistical imputation methods were applied to combine the datasets of different genotype arrays (ASA chip and KBA chip). Statistical imputation of genotypes was performed using Minimac4 software with all reference haplotypes available in the Haplotype Reference Consortium (HRC-r1.1 2016) on the University of Michigan Imputation Server. Consequently, the present inventors performed quality control (QC) after statistical imputation with (i) a MAF <1% or (ii) low imputation quality (for imputed SNPs, $R^2$ <0.8). To identify appropriate combinations of the two genotype datasets, principal component analysis (PCA) was performed using EIGENSTRAT. In addition, PCA was performed on 1000 Genomes Project samples, and two genotype datasets were projected onto a PCA plot to confirm racial distinctions. Based on the genotype data, subjects were excluded according to the following criteria: (i) call rate <95%; (ii) sex mismatch; (iii) excess heterozygosity ($\pm 5$ standard deviations [SDs] from the mean); or (iv) one of the related pairs with second-degree consanguinity or less as estimated using KING software.

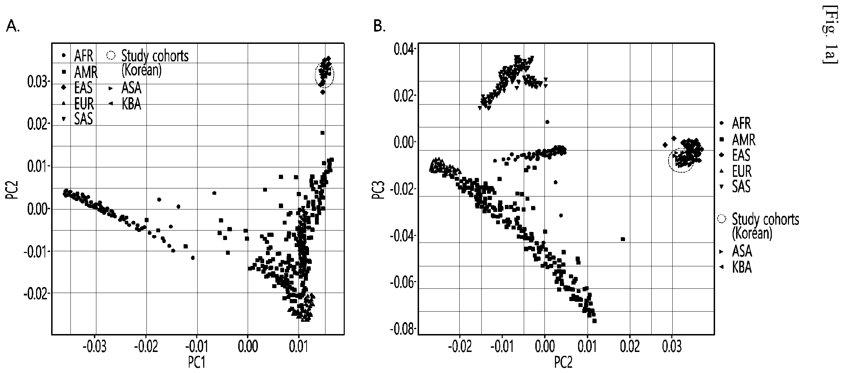

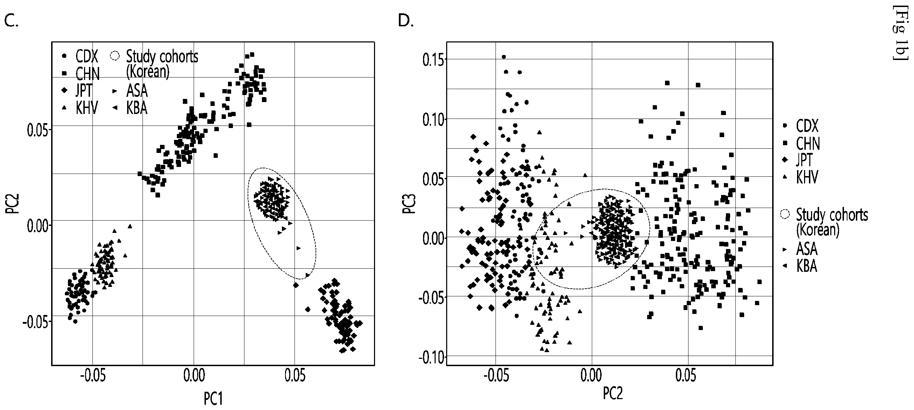

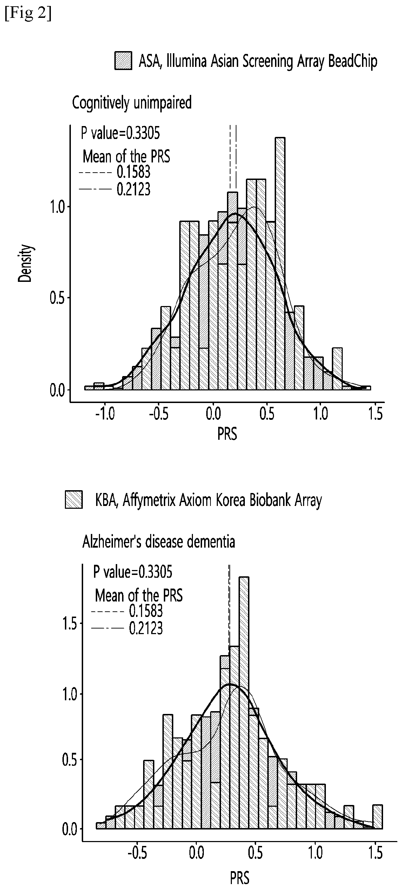

Comparison of principal components between the Korean study population and the 1,000 Genomes Project population revealed that there was racial overlap in principal component analysis (PCA) with the 1,000 Genomes Project dataset and data from other East Asian populations. However, there was no stratification by genotype array, and the distribution of polygenic risk scores (PRSs) among study subjects by genotype array did not differ significantly by genotype array (FIGS. 1A and 1B and FIG. 2).

### 4. Amyloid positron emission tomography (PET)

A portion of subjects (n = 1,214) from the validation and amnestic mild cognitive impairment (aMCI) datasets were subjected to amyloid positron emission tomography (PET), performed using a Discovery STE PET/computed tomography scanner (GE Medical Systems, Milwaukee, WI, USA). PET images were acquired for 20 minutes starting 90 minutes after intravenous injection of 18F-florbetaben or 18F-flutemetamol. amyloid $\beta$ (A$\beta$) positivity or negativity was determined by well-trained nuclear medicine physicians using visual assessments of florbetaben PET or flutemetamol PET. Positivity for tracer uptake was assessed in four cortical regions (lateral temporal, frontal, parietal, and posterior cingulate cortices) for florbetaben PET and five cortical regions (lateral temporal, frontal, parietal, posterior cingulate cortices, and striatum) for flutemetamol PET. Amyloid PET positivity was defined as having at least one cortical region with evidence of positive uptake.

### 5. GWAS Summary Statistics

To investigate the transferability of polygenic risk scores (PRSs) in the Korean population, summary statistics generated in the European International Genomics of Alzheimer's Project (IGAP) METAGWAS (11,480,632 SNPs in 21,982 AD patients and 41,944 controls) were utilized.

### 6. PRS generation

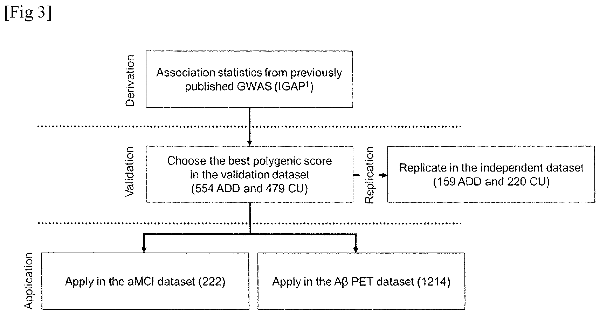

Based on the data from previous studies, 3,877 SNPs surrounding APOE (chromosome 19, 44,400 to 46,500 kb, GRCH37/hg19) were excluded to derive a PRS independent of the APOE region, and PRSice-2 was used based on the previous European IGAP GWAS summary statistics to determine the best parameters (P-value threshold and linkage disequilibrium (LD) $r^2$ value) for PRS calculation. The P values and effect sizes of summary statistics were used to generate the best PRS model on the validation dataset (554 Koreans with Alzheimer's disease dementia (ADD) and 479 cognitively unimpaired (CU) controls). To derive the best model, testing was performed by including SNPs while varying a range of P-value thresholds ($5 \times 10^{-8}$ to 1.0) of the GWAS performed by Kunkle et al. Furthermore, the linkage disequilibrium (LD) $r^2$ (0.1-0.9) range within 1,000 kb was examined to investigate the critical value showing the largest Nagelkerke $R^2$ value calculated by logistic regression. Thereafter, the same SNPs and weighted values were used to replicate the PRS associations in an independent dataset of 379 specimens (159 Alzheimer's disease dementia (ADD) cases and 220 cognitively unimpaired (CU) controls) and an applied dataset of 222 patients with amnestic mild cognitive impairment (aMCI), and an overview showing the study datasets and analysis steps was shown (FIG. 3).

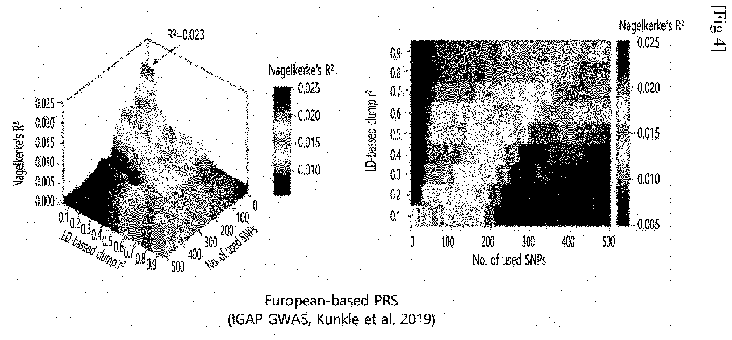

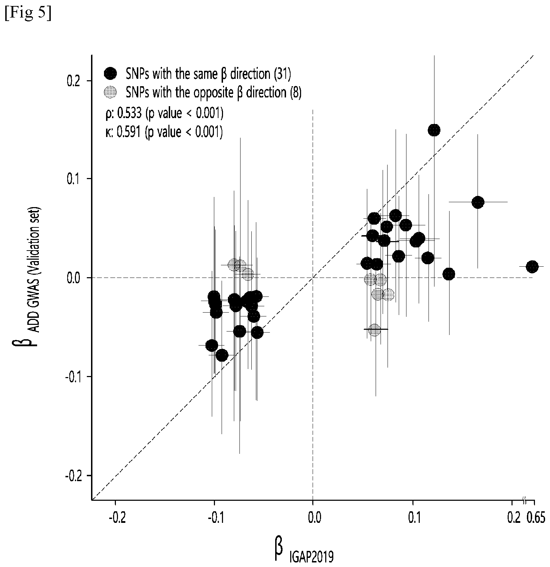

As a result, across various thresholds (P and LD values), the highest Nagelkerke $R^2$ value was identified at 0.020 (P < 0.001) for IGAP GWAS-based PRS, and the highest Nagelkerke $R^2$ value (0.020) was identified at P and LD values of $4.15 \times 10^{-6}$ and 0.1, respectively, among various thresholds (FIG. 4). Further, 39 SNPs were selected from this threshold and the beta coefficients were used for PRS generation (Table 2), confirming a high correlation (Spearman correlation = 0.533, P < 0.001) between the beta coefficients of the 39 SNPs calculated in the IGAP dataset and those in the validation dataset (FIG. 5).

[Table 2]

| CHR | SNP | Near est gene | Risk alle le | EUR[1] M AF | EAS[1] M AF | IGAP Be ta[2] | SE[2] | Korean (our validation dataset) Be ta[3] | S E[3] |
|---|---|---|---|---|---|---|---|---|---|
| 1 0 | rs12358 692 | RP1 1-1381 18.1 | T | 0 .6 5 7 2 | 0 .3 2 6 4 | 0. 64 29 | 0.0 154 | 0.0 13 3 | 0 .0 8 2 8 |
| 11 | rs11218 343 | SOR L1 | C | 0 .0 3 8 9 | 0 .2 9 7 7 | -0 .2 05 3 | 0.0 369 | -0. 09 36 | 0 .0 8 2 6 |
| 2 | rs67338 39 | BIN1 | T | 0 .3 9 2 1 | 0 .4 3 6 3 | 0. 16 93 | 0.0 154 | 0.0 04 8 | 0 .0 7 5 9 |
| 1 | rs67951 5 | CR1 | T | 0 .1 9 8 8 | 0 .0 2 8 4 | 0. 15 08 | 0.0 183 | 0.1 81 6 | 0 .2 1 8 6 |
| 1 | rs17526 84 | CR1 | A | 0 .2 0 3 7 | 0 .3 5 2 6 | 0. 14 32 | 0.0 178 | 0.0 24 1 | 0 .0 7 8 9 |
| 2 0 | rs60147 24 | CAS S4 | G | 0 .0 9 0 5 | 0 .3 6 8 9 | -0 .1 31 9 | 0.0 259 | -0. 04 74 | 0 .0 7 9 6 |
| 3 | rs68051 48 | CLE C3B | C | 0 .0 8 9 1 | 0 .0 8 1 2 | -0 .1 29 3 | 0.0 257 | -0. 04 55 | 0 .1 3 8 7 |
| 8 | rs15322 76 | CLU | T | 0 .3 9 1 9 | 0 .2 0 3 1 | -0 .1 266 | 0.0 154 | -0. 08 34 | 0 .0 9 0 1 |
| 11 | rs15827 63 | MS4 A6A | A | 0 .3 5 9 3 | 0 .1 3 3 | -0 .1 23 2 | 0.0 149 | -0. 02 15 | 0 .0 9 8 |
| 1 4 | rs17125 924 | FER MT2 | G | 0 .0 9 5 4 | 0 .2 2 6 2 | 0. 12 22 | 0.0 246 | 0.0 29 8 | 0 .0 8 8 9 |
| 2 | rs35832 505 | BIN1 | C | 0 .1 6 6 2 | 0 .0 5 6 2 | 0. 12 13 | 0.0 19 | 0.0 31 8 | 0 .1 4 1 9 |
| 1 1 | rs38511 79 | PIC ALM | T | 0 .3 5 4 2 | 0 .4 1 0 3 | -0 .1 19 8 | 0.0 148 | -0. 04 33 | 0 .0 7 8 3 |
| 2 | rs35695 568 | RPL 21P3 2 | T | 0 .0 9 6 3 | 0 .1 2 4 7 | 0. 11 52 | 0.0 247 | 0.0 64 5 | 0 .1 1 3 |
| 11 | rs56201 148 | MS4 A6A | T | 0 .3 8 2 3 | 0 .2 1 8 9 | -0 .1 13 7 | 0.0 146 | -0. 09 55 | 0 .0 9 6 5 |
| 1 9 | rs12151 021 | ABC A7 | A | 0 .3 2 0 5 | 0 .5 0 8 1 | 0. 10 71 | 0.0 169 | 0.0 26 7 | 0 .0 7 3 7 |
| 7 | rs11767 557 | EPH A1-AS1 | C | 0.2 0 1 1 | 0.1 4 0 1 | -0 .102 8 | 0.0 182 | -0. 0759 | 0 .1 0 6 8 |
| 11 | rs67472 071 | SPI1 | A | 0 .3 3 9 6 | 0 .3 0 2 6 | -0 .0 98 1 | 0.0 152 | -0. 02 81 | 0 .0 7 7 3 |
| 1 9 | rs37950 65 | ABC A7 | C | 0 .3 4 9 8 | 0 .2 5 4 8 | 0. 09 68 | 0.0 171 | -0. 01 52 | 0 .0 9 0 8 |
| 1 6 | rs37527 86 | MTS S2 | G | 0 .2 2 1 3 | 0 .1 3 2 4 | 0. 09 64 | 0.0 209 | 0.0 35 4 | 0 .1 0 3 8 |
| 8 | rs73223 431 | PTK 2B | T | 0 .3 3 5 2 | 0 .2 9 9 1 | 0. 09 36 | 0.0 153 | -0. 02 11 | 0 .0 9 0 6 |
| 1 4 | rs11623 019 | SLC 24A4 | T | 0 .1 9 3 4 | 0 .4 4 4 7 | 0. 09 13 | 0.0 174 | 0.0 61 5 | 0 .0 7 7 3 |
| 1 4 | rs12590 654 | SLC 24A4 | A | 0 .3 4 2 9 | 0 .4 6 4 | -0 .0 90 6 | 0.0 157 | -0. 06 68 | 0 .0 7 7 2 |
| 1 1 | rs11039 165 | MA DD | G | 0 .2 6 6 8 | 0 .0 2 4 4 | 0. 08 94 | 0.0 158 | -0. 01 39 | 0 .2 3 2 8 |
| 16 | rs28482 811 | GPRC5B | C | 0.1 6 | 0 .2 6 7 1 | -0.0 87 2 | 0.0189 | -0.04 43 | 0 .0 8 9 4 |
| 6 | rs31353 48 | BTN L2 | A | 0 .4 1 9 8 | 0 .3 7 0 5 | 0. 08 37 | 0.0 15 | -0. 00 26 | 0 .0 8 1 8 |

(continued)

| C H R | SNP | Near est gene | Ris k alle le | E U R [1] | E A S [1] | IGAP | | Korean (our validation dataset) | |
|---|---|---|---|---|---|---|---|---|---|
| | | | | M | M | Be | SE[2] | Be | S |
| | | | | A F | A F | ta[2] | | ta[3] | E [3] |
| 6 | rs93815 63 | AL3 5535 3.1 | C | 0 .3 5 5 | 0 .1 9 7 9 | 0. 08 21 | 0.0 148 | 0.0 29 8 | 0 .0 9 7 7 |
| 6 | rs92681 12 | TSB P1-AS1 | A | 0 .3 2 5 6 | 0 .2 6 2 9 | 0. 08 15 | 0.0 159 | -0. 02 08 | 0 .0 9 8 1 |
| 1 9 | rs38654 44 | SIGL EC2 2P | A | 0 .3 2 1 2 | 0 .1 7 3 3 | -0 .0 80 4 | 0.0 158 | 0.0 03 5 | 0 .0 9 4 7 |
| 11 | rs11230 227 | MS4 A4E | A | 0 .3 8 0 4 | 0 .2 5 8 4 | 0. 07 92 | 0.0 153 | 0.0 16 2 | 0 .0 8 4 3 |
| 6 | rs92713 75 | HLA DRB 1 | A | 0 .4 8 5 | 0 .6 2 2 6 | -0 .0 78 9 | 0.0 157 | -0. 02 61 | 0 .0 8 2 |
| 11 | rs22935 79 | PSM C3 | A | 0 .3 8 6 8 | 0 .3 7 4 3 | 0. 07 71 | 0.0 145 | -0. 06 44 | 0 .0 8 2 5 |
| 8 | rs78318 10 | GUL OP | A | 0 .5 8 6 | 0 .4 0 1 3 | -0 .0 76 5 | 0.0 146 | -0. 03 43 | 0 .0 7 9 3 |
| 5 | rs11168 036 | PFD N1 | T | 0 .4 9 1 8 | 0 .4 3 3 9 | 0. 07 54 | 0.0 143 | 0.0 73 4 | 0 .0 7 6 8 |
| 11 | rs59856 1 | SLC 25A1 P1 | A | 0 .4 8 7 3 | 0 .0 9 6 3 | 0. 07 47 | 0.0 143 | 0.0 50 8 | 0 .1 0 8 8 |
| 2 1 | rs30174 32 | ADA MTS 1 | T | 0 .3 9 6 7 | 0 .7 3 3 6 | -0 .0 73 5 | 0.0 151 | -0. 04 81 | 0 .0 8 2 5 |
| 1 7 | rs25263 78 | BZR API | A | 0 .4 5 5 1 | 0 .5 3 0 4 | 0. 07 17 | 0.0 145 | -0. 00 26 | 0 .0 7 6 4 |
| 1 9 | rs81117 08 | ELL | G | 0 .3 5 6 2 | 0 .2 5 2 3 | 0. 06 96 | 0.0 151 | 0.0 66 7 | 0 .0 8 6 9 |
| 7 | rs78057 76 | EPH A1- AS1 | A | 0 .5 2 4 8 | 0 .3 7 3 2 | -0 .0 69 5 | 0.0 148 | -0. 02 37 | 0 .0 7 7 6 |
| 6 | rs12197 146 | CD2 AP | C | 0 .4 8 7 3 | 0 .1 7 3 1 | -0 .0 67 4 | 0.0 144 | -0. 01 64 | 0 .0 9 3 3 |

[1]MAF for non-Finnish EUR and EAS samples from the Genome Count Database. (gnomAD version 2.1.1, https://gnomad.broadinstitute.org)

Statistical values were obtained from [2]a previous studies and [3]our validation dataset.

Abbreviations: IGAP, International Genomics of Alzheimer's Project; CHR, chromosomal; SNP, single-nucleotide polymorphism; SE, standard error; MAF, minor allele frequency; PRS, polygenic risk score; EUR, European; EAS, East Asian.

**7. Validation and replication of PRS for Alzheimer's disease dementia (ADD) diagnosis**

After the PRS for each subject was calculated, a logistic regression analysis was performed to determine whether the PRS derived from summary statistics for Alzheimer's disease (AD) risk based on the European population was associated with Alzheimer's disease dementia (ADD) diagnosis in the validation examination and replication datasets after correcting age, sex, years of education, APOE ε4 carrier status, and the first four principal components (PCs) of genetic ancestry using a multivariate logistic regression model. In addition, to confirm that the association between Alzheimer's disease dementia (ADD) diagnosis and the PRS differed by APOE ε4 carrier status, the same analysis was performed after stratifying subjects into APOE ε4 carrier and non-carrier groups, a PRS was developed based on previous European IGAP GWAS results, and the PRS predictive performance was compared. Odds ratio (OR) and P values were calculated using a multivariate logistic regression analysis (OR per standard deviation increase in standardized PRS).

As a result, it was confirmed that high PRS was associated with an increase in the risk of Alzheimer's disease dementia (ADD) after correcting the effects of age, sex, education, and APOE ε4 status, and that PRS was associated with the risk of Alzheimer's disease dementia (ADD) in both APOE ε4 carrier (odds ratio [OR] = 2.73, 95% CI = 1.53 to 4.97, P = 0.001) and non-carrier (OR = 1.70, 95% CI = 1.14-2.59, P = 0.01) groups. Furthermore, it was confirmed that higher PRS was significantly associated with the increased risk of amnestic mild cognitive impairment (aMCI) and amyloid β (Aβ) deposition in the brain (Table 3).

[Table 3]

| | ADD diagnosis[1] | | | aMCI diagnosis[2] | | Aβ PET deposition[3] | |
|---|---|---|---|---|---|---|---|
| Dataset | Validation | | Replication | | Application | | Application | |
| Diagnosis, no. | CU (n=479) vs. ADD (n=554) | | CU (n=220) vs. ADD (n=159) | | CU (n=479) vs. aMCI (n=220) | | Aβ (-) (n=564) vs. Aβ (+) (n=650) | |
| | OR (95% CI) | $P$ | OR (95% CI) | $P$ | OR (95% CI) | $P$ | OR (95% CI) | $P$ |
| PRS | 1.95 (1.40-2.72) | <0.001 | 1.85 (1.05-3.32) | 0.036 | 1.74 (1.16-2.64) | 0.008 | 1.81 (1.32-2.48) | <0.001 |

The methods for diagnosing Alzheimer's disease dementia (ADD), amnestic mild cognitive impairment (aMCI), and amyloid beta (Aβ) PET deposition were as follows:

[1]Diagnosis (CU=0, ADD=1).

$$= \text{sex} + \text{age} + \text{years of education} + \text{PC1-4} + \text{APOE } \varepsilon 4 \text{ carrier (0 or 1)} + \text{PRS}$$

[2]Diagnosis (CU=0, aMCI=1) confirmed

$$= \text{sex} + \text{age} + \text{years of education} + \text{PC1-4} + \text{APOE } \varepsilon 4 \text{ carrier (0 or 1)} + \text{PRS}$$

[3]Amyloid beta (Aβ) deposition (negative number = 0, positive number = 1) = sex + age + years of education + PC1-4 + APOE ε4 carrier (0 or 1) + PRS

Abbreviations: CU, cognitively unimpaired; ADD, Alzheimer's disease dementia; OR, odds ratio; CI, confidence interval; aMCI, amnestic mild cognitive impairment; Aβ, amyloid beta; PRS, polygenic risk score; PC, principal component.

**8. Application of PRS in various phenotypes**

A multivariate logistic regression analysis was performed on subjects with amnestic mild cognitive impairment (aMCI) to assess whether the PRS predicted aMCI independently of age, sex, years of education, APOE ε4 carrier status, and the first four principal components (PCs) of genetic ancestry. In a portion of subjects (n=1,214) who were also subjected to amyloid (Aβ) PET, a logistic regression analysis was also performed to assess whether the PRS predicted amyloid (Aβ) positivity, and the effects of age, sex, years of education, and APOE ε4 carrier status, which had been subjected to amyloid β (Aβ) PET, were adjusted.

Further, to test the clinical utility of the PRS, a multivariate logistic model to predict Alzheimer's disease dementia (ADD) diagnosis for each subject was developed, and an area under curve (AUC) was measured to assess the performance of the logistic model. For internal validation, 10-fold cross-validation was performed with 100 repetitions using validation data. The mean AUC with the 95% confidence interval (CI) of the models was reported, and the AUCs of models were compared by applying the DeLong test.

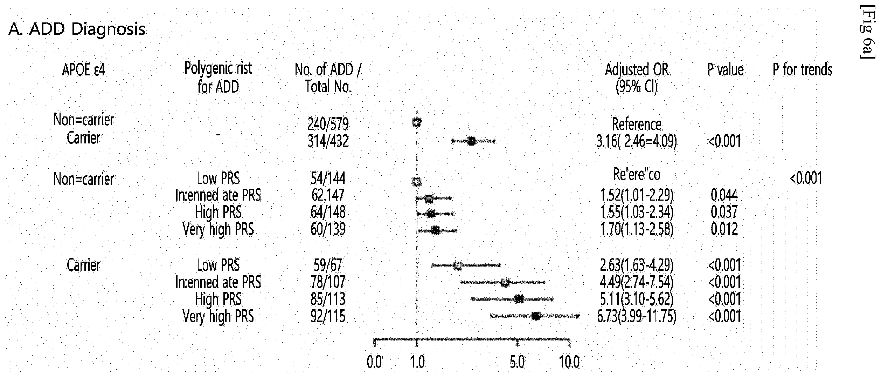

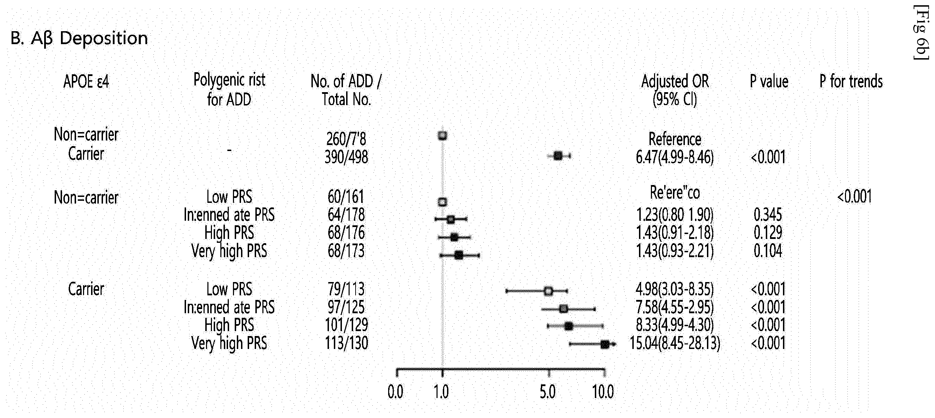

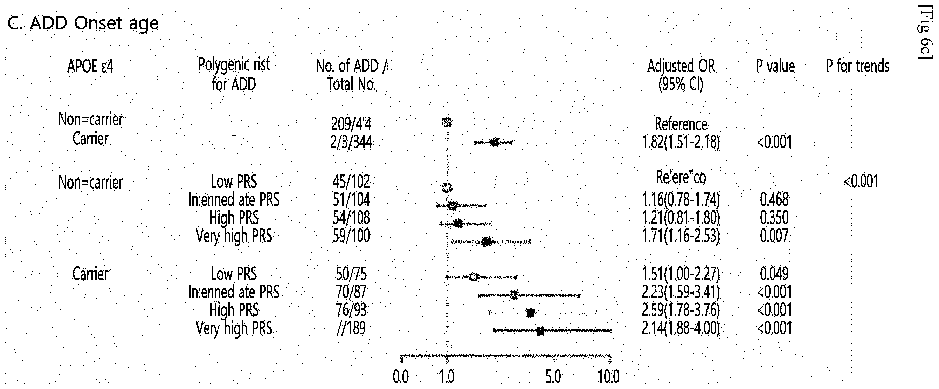

As a result, in the prediction model, the model including only clinical factors (age, sex, and years of education) (Model 1) showed an AUC of 0.581 (95% CI = 0.578 to 0.585), and it was confirmed that the predictive performance increased after integrating APOE ε4 status into Model 1 (Model 2) (AUC=0.693; 95% CI=0.690 to 0.696). In addition, it was confirmed that when the model included PRS (Model 3), the predictive performance was remarkably improved compared to Model 2 (AUC=0.706; 95% CI=0.703-0.709, P=6.30×10$^{-9}$) (FIGS. 6A to 6C).

Furthermore, subjects were stratified based on quartiles of PRS, it was evaluated whether PRS could also be used for risk stratification in addition to APOE ε4 genotype, and it was evaluated whether subjects with a higher PRS exhibited earlier development of Alzheimer's disease (AD) than those with a lower PRS. Further, Cox regression analysis was performed by employing age at last clinical visit or age at onset of Alzheimer's disease (AD) as a time variable and Alzheimer's disease (AD) as a status variable.

As a result, it was confirmed that when PRS and APOE ε4 status were combined, in both the APOE ε4 carrier and non-carrier, the risks of Alzheimer's disease dementia (ADD), amyloid β (Aβ) deposition, and early onset of Alzheimer's disease dementia increased in a stepwise manner according to PRS quartile (FIGS. 6A to 6C). In particular, compared to the APOE ε4 non-carriers in the low PRS group, the APOE ε4 carriers in the very high PRS group were confirmed to have a 6.73-fold (95% CI=3.99 to 11.75), 15.04-fold (95% CI=8.45 to 28.13), and 2.74-fold (95% CI=1.88 to 4.00) higher risk of Alzheimer's disease dementia (ADD), amyloid β (Aβ) deposition, and age at initial onset of symptoms, respectively. This coincides with previous findings showing that PRS is associated with Alzheimer's disease pathology (Aβ deposition, tau and neurodegeneration), and it was confirmed that it is important for predicting prognosis and selecting patients for clinical trials of anti-Aβ therapies to identify patients with amyloid β (Aβ) deposition. Currently, diagnostic tools for measuring amyloid β (Aβ) deposition are either invasive (cerebrospinal fluid examination) or expensive (PET). The study results highlight that genetic data (PRS and APOE ε4 status) obtained from less invasive methods (blood or saliva specimen assessment) can be used to pre-screen for amyloid β (Aβ) positivity.

In addition, it was confirmed that patients with a high PRS were more likely to develop Alzheimer's disease dementia (ADD) symptoms at a young age. The mean age at onset of symptoms was about 3.7 years younger in the very high PRS group than in the low PRS group. It is well known that APOE ε4 is associated with the onset of early symptoms of Alzheimer's disease dementia (ADD), and through the results of the present inventors, it was confirmed that PRS further accelerates the age of onset of symptoms beyond the effect of APOE ε4.

### 9. Statistical analysis

Categorical and continuous variables for demographic and clinical characteristics of subjects according to PRS quantiles are presented as counts (%) and means (SDs), respectively. P values were obtained using a chi-square test in analysis of variance for categorical and continuous variables (Table 4). Two-sided P values were reported, and a P value <0.05 was defined as statistically significant. Furthermore, all statistical analyses and results were visualized using PLINK 1.90, R version 3.6.1 (R Project for Statistical Computing) and MATLAB.

[Table 4]

| | Low PRS group (n=314) | Intermedi ate PRS group (n=314) | High PRS group (n=314) | Very high PRS group (n=313) | P |
|---|---|---|---|---|---|
| Age, mean (SD), year | 72.6±8 .9 | 72.6±8.8 | 71.9±9.1 | 71.7±8.7 | 0.14 6 |
| Education, mean (SD), year | 11.2±4 .9 | 10.9±5.0 | 10.7±5.1 | 11.1±4.9 | 0.76 1 |
| Female sex, no. (%) | 172 (54.8) | 182 (58.0) | 197 (62.7) | 188 (60.1) | 0.22 1 |
| *APOE* ε4 carrier, no. (%) | 117 (37.3) | 129 (41.1) | 132 (42.0) | 133 (42.5) | 0.53 1 |
| Amyloid positivity, no. (%) | 139 (45.7) | 161 (53.1) | 169 (55.6) | 181 (59.7) | 0.00 5 |
| Age at ADD symptom onset, mean (SD), year | 69.0±9 .9 | 68.1±9.8 | 66.5±10. 4 | 65.3±9.7 | 0.01 2 |
| Diagnosis, no. (%) | | | | | 0.00 5 |
| CU | 150 (47.8% ) | 115 (36.6%) | 112 (35.7%) | 102 (32.6%) | |
| aMCI | 51 (16.2% ) | 59 (18.8%) | 53 (16.9%) | 59 (18.8%) | |
| ADD | 113 (36.0% ) | 140 (44.6%) | 149 (47.5%) | 152 (48.6%) | |

Abbreviations: CU, cognitively unimpaired; aMCI, amnestic mild cognitive impairment; ADD, Alzheimer's disease dementia; PRS, polygenic risk score; SD, standard deviation.

### 10. Prediction model for risk group for Alzheimer's disease dementia (ADD)

### (1) Prediction model for risk group for Alzheimer's disease dementia (ADD) in consideration of coefficients for each SNP

In consideration of the actual coefficients for each SNP (Table 5), a calculation formula corresponding to the following Mathematical Formula 1 that can be used for a prediction model for the risk group was obtained.

<Model 1> PRS (39 SNPs)= rs12358692*0.6429 + rs11218343*-0.2053 + rs6733839*0.1693 + rs679515*0.1508 + rs1752684*0.1432 + rs6014724*-0.1319 + rs6805148*-0.1293 + rs1532276*-0.1266 + rs1582763*-0.1232 + rs17125924*0.1222 + rs35832505*0.1213 + rs3851179*-0.1198 + rs35695568*0.1152 + rs56201148*-0.1137 + rs12151021*0.1071 + rs11767557*-0.1028 + rs67472071*-0.0981 + rs3795065*0.0968 + rs3752786*0.0964 + rs73223431*0.0936 + rs11623019*0.0913 + [Mathematical Formula 1] rs12590654*-0.0906 + rs11039165*0.0894 + rs28482811*-0.0872 + rs3135348*0.0837 + rs9381563*0.0821 + rs9268112*0.0815 + rs3865444*-0.0804 + rs11230227*0.0792 + rs9271375*-0.0789 + rs2293579*0.0771 + rs7831810*-0.0765 + rs11168036*0.0754 + --> rs598561*0.0747 + rs3017432*-0.0735 + rs2526378*0.0717 + rs8111708*0.0696 + rs7805776*-0.0695 + rs12197146*-0.0674

Further, <Model 1> the predictive performance of the PRS model alone for the risk group for Alzheimer's disease dementia (ADD) was analyzed, and the values of the area under curve (AUC): 0.5678; Nagelkerke $R^2$: 0.0203; and P-value < 0.0001 were obtained.

[Table 5]

|   | Constituen t element | Coeffi cient |    | Constituen t element | Coef ficie nt |
|---|---|---|---|---|---|
| 1 | rs12358692 | 0.642 9 | 21 | rs11623019 | 0.09 13 |
| 2 | rs11218343 | -0.20 53 | 22 | rs12590654 | -0.0 906 |
| 3 | rs6733839 | 0.169 3 | 23 | rs11039165 | 0.08 94 |
| 4 | rs679515 | 0.150 8 | 24 | rs28482811 | -0.0 872 |
| 5 | rs1752684 | 0.143 2 | 25 | rs3135348 | 0.08 37 |
| 6 | rs6014724 | -0.13 19 | 26 | rs9381563 | 0.08 21 |
| 7 | rs6805148 | -0.12 93 | 27 | rs9268112 | 0.08 15 |
| 8 | rs1532276 | -0.12 66 | 28 | rs3865444 | -0.0 804 |
| 9 | rs1582763 | -0.12 32 | 29 | rs 11230227 | 0.07 92 |
| 10 | rs17125924 | 0.122 2 | 30 | rs9271375 | -0.0 789 |
| 11 | rs35832505 | 0.121 3 | 31 | rs2293579 | 0.07 71 |
| 12 | rs3851179 | -0.11 98 | 32 | rs7831810 | -0.0 765 |
| 13 | rs35695568 | 0.115 2 | 33 | rs11168036 | 0.07 54 |
| 14 | rs56201148 | -0.11 37 | 34 | rs598561 | 0.07 47 |
| 15 | rs12151021 | 0.107 1 | 35 | rs3017432 | -0.0 735 |
| 16 | rs11767557 | -0.10 28 | 36 | rs2526378 | 0.07 17 |
| 17 | rs67472071 | -0.09 81 | 37 | rs8111708 | 0.06 96 |
| 18 | rs3795065 | 0.096 8 | 38 | rs7805776 | -0.0 695 |
| 19 | rs3752786 | 0.096 4 | 39 | rs12197146 | -0.0 674 |
| 20 | rs73223431 | 0.0936 |    |    |    |

**(2) Prediction model for risk group for Alzheimer's disease dementia (ADD) when further including four factors (age, sex, years of education, and APOE ε4)**

When each of the four factors (age, sex, years of education, and APOE ε4) was included, a calculation formula used in the prediction model for the risk group for Alzheimer's disease dementia (ADD) was obtained. Definitions for each factor; PRS is a genetic risk score for each individual calculated by the model suggested above; age is assigned in years; sex is defined as 1 for males and 2 for females; years of education are assigned in years; and for the APOE genotype among the indicators of the individual, it is possible to further include obtaining a score for each indicator by assigning a score of 0 for ε2/ε2, ε2/ε3 and ε3/ε3 and a score of 1 for ε2/ε4, ε3/ε4 and ε3/ε4. A calculation formula corresponding to the following Mathematical Formula 2, which can be used for a regression model with PRS scores and four additional factors as dependent terms, was obtained.

PRS (including 4 factors) = <Model 1> PRS *0.66119 + age*0.02879 + sex*0.03618 + year of education*-0.02615 + APOEε4*1.37120      [Mathematical Formula 2]

In addition, the predictive performance of the PRS <Model 1>, in which the four factors were considered together, for the risk group for Alzheimer's disease dementia (ADD) was analyzed, and the values of the area under curve (AUC): 0.7101; Nagelkerke $R^2$: 0.1756; and *P*-value=0.0002 were obtained.

**(3) Predictive ability of 31 SNPs**

Model 2 is an additional selection process from 39 SNPs used in the construction of Model 1, and a PRS model was constructed by selecting 31 SNPs with the same association β coefficient direction between the Alzheimer's disease dementia (ADD) patient group and the control group from Korean data and European data (FIG. 5). The difference between the models is the difference due to the presence or absence of factors, and the estimated coefficients for each factor are the same (Table 6). Furthermore, in consideration of the coefficients for the selected 31 SNPs, a calculation formula corresponding to the following Mathematical Formula 3 that can be used for a prediction model for the risk group was obtained.

<Model 2> PRS (31 SNPs with feature selection) = rs12358692*0.6429 + rs11218343*-0.2053 + rs6733839*0.1693 + rs679515*0.1508 + rs1752684*0.1432 + rs6014724*-0.1319 + rs6805148*-0.1293 + rs1532276*-0.1266 + rs1582763*-0.1232 + rs17125924*0.1222 + rs35832505*0.1213 + rs3851179*-0.1198 + rs35695568*0.1152 + rs56201148*-0.1137 + rs12151021*0.1071 + rs11767557*-0.1028 +      [Mathematical Formula 3] rs67472071*-0.0981 + rs3752786*0.0964 + rs11623019*0.0913 + rs12590654*-0.0906 + rs28482811*-0.0872 + rs9381563*0.0821 + rs11230227*0.0792 + rs9271375*-0.0789 + rs7831810*-0.0765 + rs11168036*0.0754 + rs598561*0.0747 + rs3017432*-0.0735 + rs8111708*0.0696 + rs7805776*-0.0695 + rs12197146*-0.0674

As a result of analyzing the predictive performance of the PRS model for the risk group for Alzheimer's disease dementia (ADD), it was confirmed that the model of additionally selected 31 SNPs had a more excellent ability to predict the risk group for Alzheimer" disease dementia (ADD) than the model of 39 SNPs, and the values of the area under curve (AUC): 0.5787; Nagelkerke $R^2$: 0.02771; and P-value < 0.0001 were obtained.
As a result of analyzing the predictive performance of the PRS <Model 1>, in which the four factors were considered together, for the risk group for Alzheimer's disease dementia (ADD), the values of the area under curve (AUC): 0.7143; Nagelkerke $R^2$: 0.1804; and P-value<0.0001 were obtained.

[Table 6]

|  | Constituent element | Coefficient |  | Constituent element | Coefficient |
|---|---|---|---|---|---|
| **1** | rs12358692 | 0.6429 | **17** | rs67472071 | -0.0981 |
| **2** | rs11218343 | -0.2053 | **18** | rs3752786 | 0.0964 |
| **3** | rs6733839 | 0.1693 | **19** | rs11623019 | 0.0913 |
| **4** | rs679515 | 0.1508 | **20** | rs12590654 | -0.0906 |
| **5** | rs1752684 | 0.1432 | **21** | rs28482811 | -0.0872 |
| **6** | rs6014724 | -0.1319 | **22** | rs9381563 | 0.0821 |
| **7** | rs6805148 | -0.1293 | **23** | rs 11230227 | 0.0792 |
| **8** | rs1532276 | -0.1266 | **24** | rs9271375 | -0.0789 |
| **9** | rs1582763 | -0.1232 | **25** | rs7831810 | -0.0765 |
| **10** | rs17125924 | 0.1222 | **26** | rs11168036 | 0.0754 |
| **11** | rs35832505 | 0.1213 | **27** | rs598561 | 0.0747 |
| **12** | rs3851179 | -0.1198 | **28** | rs3017432 | -0.0735 |

(continued)

|    | Constituent element | Coefficient |    | Constituent element | Coefficient |
|----|---------------------|-------------|----|---------------------|-------------|
| 13 | rs35695568          | 0.1152      | 29 | rs8111708           | 0.0696      |
| 14 | rs56201148          | -0.1137     | 30 | rs7805776           | -0.0695     |
| 15 | rs12151021          | 0.1071      | 31 | rs12197146          | -0.0674     |
| 16 | rs11767557          | -0.1028     |    |                     |             |

**(4) Performance of 28 PRS models**

The performance of the PRS models constructed by sequentially excluding 39 SNPs (while reducing ones in a less significant sequence according to the significance level of *P*-value) and 28 types of PRS models were obtained.

In the experiment, several PRS models were constructed while sequentially removing SNPs with relatively high significance levels (*P*-values) from the PRS (39 SNPs) model, each model was evaluated alone or as a model in which four well-known factors (age, sex, years of education, and APOE $\varepsilon$4) were considered, and performance levels were compared with the AUC and significance level *P*-value.

As a result of the experiment, it was confirmed that a total of 28 detailed PRS models, from the PRS (38 SNPs) model in which one SNP was excluded from the <Model 1> PRS to the PRS using 11 SNPs, acted as factors which predict a risk group for Alzheimer's disease dementia (ADD) while securing statistical significance (Table 7).

[Table 7]

| No. | SNP | Nearest Gene | Risk allele | Beta (IGAP2019) | Association *P*-value (IGAP2019) | AUC performance and PRS significance level of PRS single model | Final model AUC performanc e and significance level of PRS after correction of five factors |
|-----|-----|-------------|------------|-----------------|----------------------------------|----------------------------------------------------------------|---------------------------------------------------------------------------------------------|
| 1 | rs6733839 | *BIN1* | T | 0.1693 | 4.02E-28 | AUC=0.5043 , P=0.8136 | AUC=0.697 8, P=0.8888 |
| 2 | rs1582763 | *MS4A6A* | A | -0.1232 | 1.19E-16 | AUC=0.5089 , P=0.4870 | AUC=0.698 2, P=0.6347 |
| 3 | rs679515 | *CR1* | T | 0.1508 | 1.56E-16 | AUC=0.5191 , P=0.2041 | AUC=0.698 8, P=0.3380 |
| 4 | rs1532276 | *CLU* | T | -0.1266 | 1.73E-16 | AUC=0.5285 , P=0.0738 | AUC=0.700 1, P=0.1627 |
| 5 | rs3851179 | *PICALM* | T | -0.1198 | 5.81E-16 | AUC=0.5279 , P=0.0853 | AUC=0.701 2, P=0.1847 |
| 6 | rs1752684 | *CR1* | A | 0.1432 | 9.39E-16 | AUC=0.5363 , P=0.0304 | AUC=0.703 1, P=0.0828 |
| 7 | rs56201148 | *MS4A6A* | T | -0.1137 | 6.00E-15 | AUC=0.5370 , P=0.0276 | AUC=0.703 3, P=0.0586 |
| 8 | rs67472071 | *SPI1* | A | -0.0981 | 1.14E-10 | AUC=0.5392 , P=0.0293 | AUC=0.703 3, P=0.0639 |
| 9 | rs35832505 | *BIN1* | T | -0.1213 | 1.82E-10 | AUC=0.5393 , P=0.0260 | AUC=0.703 6, P=0.0501 |
| 10 | rs12151021 | *ABCA7* | A | 0.1071 | 2.56E-10 | AUC=0.5386 , P=0.0339 | AUC=0.703 7, P=0.0547 |

(continued)

| No. | SNP | Nearest Gene | Risk allele | Beta (IGAP2019 ) | Association *P*-value (IGAP2019) | AUC performance and PRS significance level of PRS single model | Final model AUC performanc e and significance level of PRS after correction of five factors |
|---|---|---|---|---|---|---|---|
| 11 | rs73223431 | PTK2B | T | 0.0936 | 8.34E-10 | AUC=0.5463 , P=0.0135 | AUC=0.704 6, P=0.0298 |
| 12 | rs12590654 | SLC24A4 | A | -0.0906 | 8.73E-09 | AUC=0.5465 , P=0.0132 | AUC=0.704 5, P=0.0307 |
| 13 | rs11039165 | MADD | A | -0.0894 | 1.50E-08 | AUC=0.5504 , P=0.0068 | AUC=0.705 7, P=0.0152 |
| 14 | rs11767557 | EPHAI-AS1 | T | 0.1028 | 1.56E-08 | AUC=0.5452 , P=0.0126 | AUC=0.704 3, P=0.0265 |
| 15 | rs3795065 | ABCA7 | T | -0.0968 | 1.63E-08 | AUC=0.5442, P=0.0140 | AUC=0.7045, P=0.0269 |
| 16 | rs3135348 | BTNL2 | A | 0.0837 | 2.34E-08 | AUC=0.5485 , P=0.0025 | AUC=0.706 0, P=0.0074 |
| 17 | rs11218343 | SORL1 | T | 0.2053 | 2.63E-08 | AUC=0.5495 , P=0.0025 | AUC=0.705 8, P=0.0073 |
| 18 | rs9381563 | AL355353. 1 | T | -0.0821 | 2.93E-08 | AUC=0.5454 , P=0.0056 | AUC=0.704 6, P=0.0112 |
| 19 | rs12358692 | RP11-138I18.1 | T | 0.6429 | 4.65E-08 | AUC=0.5528 , P=0.0022 | AUC=0.706 3, P=0.0044 |
| 20 | rs2293579 | PSMC3 | A | 0.0771 | 1.14E-07 | AUC=0.5537 , P=0.0021 | AUC=0.706 0, P=0.0053 |
| 21 | rs11168036 | PFDN1 | T | 0.0754 | 1.43E-07 | AUC=0.5591 , P=0.0008 | AUC=0.707 7, P=0.0022 |
| 22 | rs7831810 | GULOP | A | -0.0765 | 1.58E-07 | AUC=0.5594 , P=0.0006 | AUC=0.707 8, P=0.0020 |
| 23 | rs11623019 | SLC24A4 | T | 0.0913 | 1.66E-07 | AUC=0.5570 , P=0.0008 | AUC=0.707 3, P=0.0023 |
| 24 | rs598561 | SLC25A1P 1 | A | 0.0747 | 1.87E-07 | AUC=0.5561 , P=0.0011 | AUC=0.706 5, P=0.0032 |
| 25 | rs 11230227 | MS4A4E | A | 0.0792 | 2.27E-07 | AUC=0.563, P=0.0004 | AUC=0.707 7, P=0.0013 |
| 26 | rs9268112 | TSBPI-AS1 | A | 0.0815 | 2.94E-07 | AUC=0.5624 , P=0.0005 | AUC=0.707 8, P=0.0016 |
| 27 | rs6014724 | CASS4 | A | 0.1319 | 3.65E-07 | AUC=0.5624 , P=0.0004 | AUC=0.707 7, P=0.0013 |
| 28 | rs3865444 | SIGLEC22 P | A | -0.0804 | 3.93E-07 | AUC=0.5619 , P=0.0003 | AUC=0.707 4, P=0.0011 |
| 29 | rs9271375 | HLA-DRBI | A | -0.0789 | 4.73E-07 | AUC=0.5628 , P=0.0003 | AUC=0.708 3, P=0.0008 |

(continued)

| No. | SNP | Nearest Gene | Risk allele | Beta (IGAP2019 ) | Association *P*-value (IGAP2019) | AUC performance and PRS significance level of PRS single model | Final model AUC performanc e and significance level of PRS after correction of five factors |
|---|---|---|---|---|---|---|---|
| 30 | rs6805148 | CLEC3B | A | 0.1293 | 4.77E-07 | AUC=0.5608 , P=0.0003 | AUC=0.707 6, P=0.0009 |
| 31 | rs17125924 | FERAMT2 | A | -0.1222 | 6.62E-07 | AUC=0.5636 , P=0.0002 | AUC=0.707 9, P=0.0005 |
| 32 | rs2526378 | BZRAP1 | A | 0.0717 | 7.74E-07 | AUC=0.5634 , P=0.0002 | AUC=0.708 4, P=0.0004 |
| 33 | rs3017432 | ADAMTS1 | T | -0.0735 | 1.05E-06 | AUC=0.5642 , P=0.0002 | AUC=0.708 9, P=0.0003 |
| 34 | rs7805776 | EPHAI-AS1 | A | -0.0695 | 2.61E-06 | AUC=0.5658 , P<0.0001 | AUC=0.708 9, P=0.0003 |
| 35 | rs12197146 | CD2AP | T | 0.0674 | 2.85E-06 | AUC=0.5646 , P<0.0001 | AUC=0.708 8, P=0.0003 |
| 36 | rs35695568 | RPL21P32 | T | 0.1152 | 3.20E-06 | AUC=0.5672 , P<0.0001 | AUC=0.709 6, P=0.0002 |
| 37 | rs28482811 | GPRCSB | T | 0.0872 | 3.94E-06 | AUC=0.5696 , P<0.0001 | AUC=0.710 1, P=0.0001 |
| 38 | rs8111708 | ELL | A | -0.0696 | 3.95E-06 | AUC=0.5680 , P<0.0001 | AUC=0.709 9, P=0.0002 |
| Following <Model 1> PRS (39 SNPs) | | | | | | | |
| 39 | rs3752786 | MTSS2 | A | -0.0964 | 4.07E-06 | AUC=0.5678 , P<0.0001 | AUC=0.710 1, P=0.0002 |

A calculation formula corresponding to the following Mathematical Formula 4 that can be used for a prediction model for the risk group was obtained.

[Mathematical Formula 4]

<Model 3> PRS (11 SNPs)

$$= rs6733839*0.1693 + rs1582763*-0.1232 + rs679515*0.1508 + rs1532276*-0.1266$$

$$+ \quad rs3851179*-0.1198 \quad + \quad rs1752684*0.1432 \quad + \quad rs56201148*-0.1137 \quad +$$

$$rs67472071*-0.0981 + rs35832505*-0.1213 + rs12151021*0.1071 + rs73223431*0.0936$$

It is possible to construct up to the following <Model 30> PRS while adding genetic factors one by one to the above mathematical formula. For example, Models 4 to 30 may be constructed as follows.

<Model 4> PRS (11 SNPs)

It is possible to construct models sequentially from = rs6733839*0.1693 + rs1582763*-0.1232 + rs679515*0.1508 + rs1532276*-0.1266 + rs3851179*-0.1198 + rs1752684*0.1432 + rs56201148*-0.1137 + rs67472071*-0.0981 + rs35832505*-0.1213 + rs12151021*0.1071 + rs73223431*0.0936 + rs12590654*-0.0906,

<Model 30> PRS (38 SNPs)

to = rs6733839*0.1693 + rs1582763*-0.1232 + rs679515*0.1508 + rs1532276*-0.1266 + rs3851179*-0.1198 + rs1752684*0.1432 + rs56201148*-0.1137 + rs67472071*-0.0981 + rs35832505*-0.1213 + rs12151021*0.1071 + rs73223431*0.0936 + rs12590654*-0.0906 + rs11039165*-0.0894 + rs11767557*0.1028 + --> rs3795065*-0.0968 + rs3135348*0.0837 + rs11218343*0.2053 + rs9381563*-0.0821 + rs12358692*0.6429 + rs2293579*0.0771 + rs11168036*0.0754 + rs7831810*-0.0765 + rs11623019*0.0913 + rs598561*0.0747 + rs11230227*0.0792 + rs9268112*0.0815 + rs6014724*0.1319 + rs3865444*-0.0804 + rs9271375*-0.0789 + rs6805148*0.1293 + rs17125924*-0.1222 + rs2526378*0.0717 + rs3017432*-0.0735 + rs7805776*-0.0695 + rs12197146*0.0674 + rs35695568*0.1152 + rs28482811*0.0872 + rs8111708*-0.0696.

## Claims

1. A method for providing information for predicting a risk group for developing Alzheimer's disease dementia or a risk group for early onset of Alzheimer's symptoms, the method comprising: bringing a sample isolated from an individual in contact with a preparation capable of identifying the presence of risk alleles of a plurality of single-nucleotide polymorphisms (SNPs); and

   determining the presence or absence of risk alleles of a plurality of single-nucleotide polymorphisms in the sample,
   wherein the plurality of single-nucleotide polymorphisms comprise rs6733839, rs1582763, rs679515, rs1532276, rs3851179, rs1752684, rs56201148, rs67472071, rs35832505, rs12151021, and rs73223431.

2. The method of claim 1, wherein the plurality of single-nucleotide polymorphisms further comprise one or more single-nucleotide polymorphisms selected from the group consisting of rs12358692, rs11218343, rs6014724, rs6805148, rs17125924, rs35695568, rs11767557, rs3795065, rs3752786, rs11623019, rs12590654, rs11039165, rs28482811, rs3135348, rs9381563, rs9268112, rs3865444, rs11230227, rs9271375, rs2293579, rs7831810, rs11168036, rs598561, rs3017432, rs2526378, rs8111708, rs7805776, and rs12197146.

3. The method of claim 1 or 2, further comprising obtaining a score for a single-nucleotide polymorphism by assigning a score of 1 to a single-nucleotide polymorphism determined to indicate the presence of a risk allele in the sample among the plurality of single-nucleotide polymorphisms, wherein among the plurality of single-nucleotide polymorphisms, a single-nucleotide polymorphism determined to be absent in the sample is assigned a score of 0.

4. The method of claim 3, further comprising obtaining a first polygenic risk score (PRS) value by multiplying the assigned score for the single-nucleotide polymorphism by a coefficient (β) assigned for each of the following single-nucleotide polymorphisms, and adding all the multiplied values,
   wherein the coefficient of rs6733839 is 0.1693, the coefficient of rs1582763 is -0.1232, the coefficient of rs679515 is 0.1508, the coefficient of rs1532276 is -0.1266, the coefficient of rs3851179 is -0.1198, the coefficient of rs1752684 is 0.1432, the coefficient of rs56201148 is -0.1137, the coefficient of rs67472071 is -0.0981, the coefficient of rs35832505 is 0.1213, the coefficient of rs12151021 is 0.1071, the coefficient of rs73223431 is 0.0936, the coefficient of rs12358692 is 0.6429, the coefficient of rs11218343 is -0.2053, the coefficient of rs6014724 is -0.1319, the coefficient of rs17125924 is 0.1222, the coefficient of rs35695568 is 0.1152, the coefficient of rs11767557 is -0.1028, the coefficient of rs3795065 is 0.0968, the coefficient of rs3752786 is 0.0964, the coefficient of rs11623019 is 0.0913, the coefficient of rs12590654 is -0.0906, the coefficient of rs11039165 is 0.0894, the coefficient of rs28482811 is -0.0872, the coefficient of rs3135348 is 0.0837, the coefficient of rs9381563 is 0.0821, the coefficient of rs9268112 is 0.0815, the coefficient of rs3865444 is -0.0804, the coefficient rs11230227 is 0.0792, the coefficient of rs9271375 is -0.0789, the coefficient of rs2293579 is 0.0771, the coefficient of rs7831810 is -0.0765, the coefficient of rs11168036 is 0.0754, the coefficient of rs598561 is 0.0747, the coefficient of rs3017432 is -0.0735, the coefficient of rs2526378 is 0.0717, the coefficient of rs8111708 is 0.0696, the coefficient of rs7805776 is -0.0695, and the coefficient of rs12197146 is -0.0674.

5. The method of claim 4, further comprising determining that, when the first PRS value is higher than the first PRS value of an individual not having Alzheimer's disease dementia, the individual is in a high risk group for developing Alzheimer's disease dementia or in a high risk group for early onset of Alzheimer's symptoms.

6. The method of claim 5, further comprising identifying one or more indicators selected from the group consisting of the individual's age, sex, years of education, and APOE genotype.

7. The method of claim 6, further comprising obtaining a score for each indicator by assigning a score based on a number of years in the case of the age and years of education among the indicators of the individual,

   assigning a score of 1 for males and a score of 2 for females in the case of sex among the indicators of the individual, and
   assigning a score of 0 for $\varepsilon2/\varepsilon2$, $\varepsilon2/\varepsilon3$, and $\varepsilon3/\varepsilon3$ and a score of 1 for $\varepsilon2/\varepsilon4$, $\varepsilon3/\varepsilon4$, and $\varepsilon4/\varepsilon4$ in the case of APOE genotype among the indicators of the individual.

8. The method of claim 7, further comprising obtaining a second PRS value by multiplying the assigned score for each indicator by a coefficient ($\beta$) assigned for each of the following indicators, and adding the first PRS value and a coefficient ($\beta$) assigned for the following first PRS value to the multiplied values,
   wherein the coefficient of the age is 0.02879, the coefficient of the sex is 0.03618, the coefficient of the year of education is -0.02615, the coefficient of the APOE genotype is 1.3712, and the coefficient of the first PRS value is 0.66119.

9. The method of claim 8, further comprising determining that, when the second PRS value is higher than the second PRS value of an individual not having Alzheimer's disease dementia, the individual is in a high risk group for developing Alzheimer's disease dementia or in a high risk group for early onset of Alzheimer's symptoms.

10. A method for providing information for predicting a risk group for developing Alzheimer's disease dementia or a risk group for early onset of Alzheimer's symptoms, the method comprising: bringing a sample isolated from an individual in contact with a preparation capable of identifying the presence of risk alleles of a plurality of single-nucleotide polymorphisms (SNPs); and

    determining the presence or absence of risk alleles of the plurality of single-nucleotide polymorphisms,
    wherein the plurality of single-nucleotide polymorphisms comprise rs6733839, rs1582763, rs679515, rs1532276, rs3851179, rs1752684, rs56201148, rs67472071, rs35832505, rs12151021, rs12358692, rs11218343, rs6014724, rs6805148, rs17125924, rs35695568, rs11767557, rs3752786, rs11623019, rs12590654, rs28482811, rs9381563, rs11230227, rs9271375, rs7831810, rs11168036, rs598561, rs3017432, rs8111708, rs7805776, and rs12197146.

11. The method of claim 1 or 10, wherein the preparation is selected from the group consisting of a primer, a probe, an aptamer, an antibody, a peptide, and combinations thereof, capable of specifically binding to a base sequence comprising the single-nucleotide polymorphism or a protein encoded by the base sequence.

12. A composition for predicting a risk group for developing Alzheimer's disease dementia or a risk group for early onset of Alzheimer's symptoms, comprising a preparation capable of confirming the presence or absence of risk alleles of a plurality of single-nucleotide polymorphisms (SNPs) in a sample isolated from an individual,
    wherein the plurality of single-nucleotide polymorphisms are rs6733839, rs1582763, rs679515, rs1532276, rs3851179, rs1752684, rs56201148, rs67472071, rs35832505, rs12151021, and rs73223431.

13. The composition of claim 12, wherein the preparation is selected from the group consisting of a primer, a probe, an aptamer, an antibody, a peptide, and combinations thereof, capable of specifically binding to a base sequence comprising the single-nucleotide polymorphism or a protein.

14. A kit for predicting a risk group for developing Alzheimer's disease dementia or a risk group for early onset of Alzheimer's symptoms, comprising the composition of claim 12.

15. A method for providing information for predicting a risk group for developing amnestic mild cognitive impairment (aMCI), the method comprising: bringing a sample isolated from an individual in contact with a preparation capable of identifying the presence of risk alleles of a plurality of single-nucleotide polymorphisms (SNPs); and

determining the presence or absence of risk alleles of the plurality of single-nucleotide polymorphisms, wherein the plurality of single-nucleotide polymorphisms comprise rs6733839, rs1582763, rs679515, rs1532276, rs3851179, rs1752684, rs56201148, rs67472071, rs35832505, rs12151021, and rs73223431.

16. A composition for predicting a risk group for developing amnestic mild cognitive impairment, comprising a preparation capable of confirming the presence or absence of risk alleles of a plurality of single-nucleotide polymorphisms (SNPs) in a sample isolated from an individual, wherein the plurality of single-nucleotide polymorphisms are rs6733839, rs1582763, rs679515, rs1532276, rs3851179, rs1752684, rs56201148, rs67472071, rs35832505, rs12151021, and rs73223431.

17. A kit for predicting a risk group for developing amnestic mild cognitive impairment, comprising the composition of claim 15.

18. A method for providing information for predicting a positron emission tomography (PET)-positive risk group for amyloid β deposition, the method comprising: bringing a sample isolated from an individual in contact with a preparation capable of identifying the presence of risk alleles of a plurality of single-nucleotide polymorphisms (SNPs); and

determining the presence or absence of risk alleles of the plurality of single-nucleotide polymorphisms, wherein the plurality of single-nucleotide polymorphisms comprise rs6733839, rs1582763, rs679515, rs1532276, rs3851179, rs1752684, rs56201148, rs67472071, rs35832505, rs12151021, and rs73223431.

19. A composition for predicting a positron emission tomography (PET)-positive risk group for amyloid β deposition, comprising a preparation capable of confirming the presence or absence of risk alleles of a plurality of single-nucleotide polymorphisms (SNPs) in a sample isolated from an individual, wherein the plurality of single-nucleotide polymorphisms are rs6733839, rs1582763, rs679515, rs1532276, rs3851179, rs1752684, rs56201148, rs67472071, rs35832505, rs12151021, and rs73223431.

20. A kit for predicting a positron emission tomography (PET)-positive risk group for amyloid β deposition, comprising the composition of claim 18.

21. A use of a preparation capable of confirming the presence or absence of risk alleles of a plurality of single-nucleotide polymorphisms (SNPs) in a sample isolated from an individual for predicting a risk group for developing Alzheimer's disease dementia or a risk group for early onset of Alzheimer's symptoms, wherein the plurality of single-nucleotide polymorphisms are rs6733839, rs1582763, rs679515, rs1532276, rs3851179, rs1752684, rs56201148, rs67472071, rs35832505, rs12151021, and rs73223431.

22. A use of a preparation capable of confirming the presence or absence of risk alleles of a plurality of single-nucleotide polymorphisms (SNPs) in a sample isolated from an individual for predicting a risk group for developing amnestic mild cognitive impairment, wherein the plurality of single-nucleotide polymorphisms are rs6733839, rs1582763, rs679515, rs1532276, rs3851179, rs1752684, rs56201148, rs67472071, rs35832505, rs12151021, and rs73223431.

23. A use of a preparation capable of confirming the presence or absence of risk alleles of a plurality of single-nucleotide polymorphisms (SNPs) in a sample isolated from an individual for predicting a positron emission tomography (PET)-positive risk group for amyloid β deposition, wherein the plurality of single-nucleotide polymorphisms are rs6733839, rs1582763, rs679515, rs1532276, rs3851179, rs1752684, rs56201148, rs67472071, rs35832505, rs12151021, and rs73223431.

[Fig. 1a]

[Fig 1b]

[Fig 2]

Cognitively unimpaired

KBA, Affymetrix Axiom Korea Biobank Array

Alzheimer's disease dementia

[Fig 3]

[Fig 4]

[Fig 5]

## A. ADD Diagnosis

| APOE ε4 | Polygenic rist for ADD | No. of ADD / Total No. | | Adjusted OR (95% CI) | P value | P for trends |
|---|---|---|---|---|---|---|
| Non=carrier | | 240/579 | | Reference | | |
| Carrier | - | 314/432 | | 3.16( 2.46=4.09) | <0.001 | |
| Non=carrier | Low PRS | 54/144 | | Re'ere"co | | <0.001 |
| | Intenned ate PRS | 62.147 | | 1.52(1.01-2.29) | 0.044 | |
| | High PRS | 64/148 | | 1.55(1.03-2.34) | 0.037 | |
| | Very high PRS | 60/139 | | 1.70(1.13-2.58) | 0.012 | |
| Carrier | Low PRS | 59/67 | | 2.63(1.63-4.29) | <0.001 | |
| | Intenned ate PRS | 78/107 | | 4.49(2.74-7.54) | <0.001 | |
| | High PRS | 85/113 | | 5.11(3.10-5.62) | <0.001 | |
| | Very high PRS | 92/115 | | 6.73(3.99-11.75) | <0.001 | |

0.0    1.0    5.0    10.0

B. Aβ Deposition

| APOE ε4 | Polygenic rist for ADD | No. of ADD / Total No. | | Adjusted OR (95% CI) | P value | P for trends |
|---|---|---|---|---|---|---|
| Non=carrier | - | 260/7'8 | | Reference | | |
| Carrier | - | 390/498 | | 6.47(4.99-8.46) | <0.001 | |
| Non=carrier | Low PRS | 60/161 | | Re'ere"co | | <0.001 |
| | In:enned ate PRS | 64/178 | | 1.23(0.80 1.90) | 0.345 | |
| | High PRS | 68/176 | | 1.43(0.91-2.18) | 0.129 | |
| | Very high PRS | 68/173 | | 1.43(0.93-2.21) | 0.104 | |
| Carrier | Low PRS | 79/113 | | 4.98(3.03-8.35) | <0.001 | |
| | In:enned ate PRS | 97/125 | | 7.58(4.55-2.95) | <0.001 | |
| | High PRS | 101/129 | | 8.33(4.99-4.30) | <0.001 | |
| | Very high PRS | 113/130 | | 15.04(8.45-28.13) | <0.001 | |

0.0  1.0  5.0  10.0

[Fig 6b]

EP 4 495 258 A1

[Fig 6c]

EP 4 495 258 A1

## C. ADD Onset age

| APOE ε4 | Polygenic rist for ADD | No. of ADD / Total No. | | Adjusted OR (95% CI) | P value | P for trends |
|---|---|---|---|---|---|---|
| Non=carrier | - | 209/4'4 | | Reference | | |
| Carrier | - | 2/3/344 | | 1.82(1.51-2.18) | <0.001 | |
| | | | | | | |
| Non=carrier | Low PRS | 45/102 | | Re'ere"co | | <0.001 |
| | In:enned ate PRS | 51/104 | | 1.16(0.78-1.74) | 0.468 | |
| | High PRS | 54/108 | | 1.21(0.81-1.80) | 0.350 | |
| | Very high PRS | 59/100 | | 1.71(1.16-2.53) | 0.007 | |
| | | | | | | |
| Carrier | Low PRS | 50/75 | | 1.51(1.00-2.27) | 0.049 | |
| | In:enned ate PRS | 70/87 | | 2.23(1.59-3.41) | <0.001 | |
| | High PRS | 76/93 | | 2.59(1.78-3.76) | <0.001 | |
| | Very high PRS | //189 | | 2.14(1.88-4.00) | <0.001 | |

0.0    1.0    5.0    10.0

## INTERNATIONAL SEARCH REPORT

International application No.

**PCT/KR2022/021602**

| A. | CLASSIFICATION OF SUBJECT MATTER |
|---|---|

**C12Q 1/6883**(2018.01)i

According to International Patent Classification (IPC) or to both national classification and IPC

| B. | FIELDS SEARCHED |
|---|---|

Minimum documentation searched (classification system followed by classification symbols)

C12Q 1/6883(2018.01); A61K 31/7088(2006.01); C12N 15/09(2006.01); C12Q 1/6869(2018.01); G01N 33/53(2006.01); G16B 30/00(2019.01); G16H 50/20(2018.01); G16H 50/30(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 알츠하이머(alzheimer), 단일염기다형성(single nucleotide polymorphism, SNP), 기억상실형 경도인지장애(aMCI: amnestic mild cognitive impairment), rs6733839, rs1582763, rs679515, rs1532276, rs3851179, rs1752684, rs56201148, rs67472071, rs35832505, rs12151021, rs73223431

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 2012-531195 A (UNIV COLLEGE CARDIFF CONSULTANTS LTD.) 10 December 2012 (2012-12-10)<br>    See claims 1 and 5. | 1-23 |
| A | ROJAS, I. D. et al. Common variants in Alzheimer's disease: Novel association of six genetic variants with AD and risk stratification by polygenic risk scores. medRxiv. 2020 (publication: 14 January 2020).<br>    Retrieved from (URL: https://doi.org/10.1101/19012021).<br>    See abstract, and Table 2. | 1-23 |
| A | US 2020-0027557 A1 (HUMAN LONGEVITY, INC.) 23 January 2020 (2020-01-23)<br>    See claims 1-23. | 1-23 |
| A | KR 10-2019-0119859 A (SAMSUNG LIFE PUBLIC WELFARE FOUNDATION et al.) 23 October 2019 (2019-10-23)<br>    See paragraphs [0026] and [0027]. | 1-23 |

| ✓ Further documents are listed in the continuation of Box C. | ✓ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **16 June 2023** | **20 June 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2022/021602**

| C. | DOCUMENTS CONSIDERED TO BE RELEVANT | |
|---|---|---|
| Category* | Citation of document, with indication, where appropriate, of the relevant passages | Relevant to claim No. |
| A | KR 10-2020-0130165 A (MJ BRAIN BIO CO., LTD.) 18 November 2020 (2020-11-18)<br>See claims 1 and 5. | 1-23 |
| A | JP 2003-289881 A (CHUBU NATIONAL HOSPITAL et al.) 14 October 2003 (2003-10-14)<br>See claims 1, 22 and 28. | 1-23 |

Form PCT/ISA/210 (second sheet) (July 2022)

INTERNATIONAL SEARCH REPORT
Information on patent family members

International application No.

**PCT/KR2022/021602**

| Patent document cited in search report | | | Publication date (day/month/year) | Patent family member(s) | | | Publication date (day/month/year) |
|---|---|---|---|---|---|---|---|
| JP | 2012-531195 | A | 10 December 2012 | AU | 2010-267835 | A1 | 15 December 2011 |
| | | | | AU | 2010-267835 | A1 | 06 January 2011 |
| | | | | AU | 2010-267835 | B2 | 06 November 2014 |
| | | | | AU | 2010-267835 | C1 | 26 February 2015 |
| | | | | AU | 2014-265063 | A1 | 11 December 2014 |
| | | | | AU | 2014-265063 | B2 | 02 February 2017 |
| | | | | CA | 2766876 | A1 | 06 January 2011 |
| | | | | CA | 2766876 | C | 28 January 2020 |
| | | | | CN | 102471800 | A | 23 May 2012 |
| | | | | CN | 103409511 | A | 27 November 2013 |
| | | | | EP | 2449134 | A2 | 09 May 2012 |
| | | | | EP | 2449134 | B1 | 27 July 2016 |
| | | | | EP | 2891723 | A1 | 08 July 2015 |
| | | | | EP | 2891723 | B1 | 04 October 2017 |
| | | | | GB | 0911539 | D0 | 12 August 2009 |
| | | | | GB | 0913357 | D0 | 16 September 2009 |
| | | | | GB | 0917326 | D0 | 18 November 2009 |
| | | | | HK | 1169455 | A1 | 25 January 2013 |
| | | | | JP | 2014-012007 | A | 23 January 2014 |
| | | | | JP | 5917395 | B2 | 11 May 2016 |
| | | | | JP | 5917460 | B2 | 18 May 2016 |
| | | | | NZ | 596614 | A | 30 November 2012 |
| | | | | US | 2012-0122703 | A1 | 17 May 2012 |
| | | | | US | 2013-0288906 | A1 | 31 October 2013 |
| | | | | US | 2015-0119264 | A1 | 30 April 2015 |
| | | | | WO | 2011-001135 | A2 | 06 January 2011 |
| US | 2020-0027557 | A1 | 23 January 2020 | WO | 2019-169049 | A1 | 06 September 2019 |
| KR | 10-2019-0119859 | A | 23 October 2019 | KR | 10-2303638 | B1 | 23 September 2021 |
| | | | | WO | 2019-199105 | A1 | 17 October 2019 |
| KR | 10-2020-0130165 | A | 18 November 2020 | WO | 2020-231081 | A1 | 19 November 2020 |
| JP | 2003-289881 | A | 14 October 2003 | US | 2004-0234990 | A1 | 25 November 2004 |
| | | | | WO | 03-012141 | A1 | 13 February 2003 |
| | | | | WO | 0301-2141 | A1 | 13 February 2003 |

Form PCT/ISA/210 (patent family annex) (July 2022)